(19) Europäisches Patentamt
European Patent Office
Office européen des brevets

(11) Publication number: **0 050 684**
**B1**

(12) **EUROPEAN PATENT SPECIFICATION**

(45) Date of publication of patent specification: **22.01.86**

(21) Application number: **80106587.1**

(22) Date of filing: **27.10.80**

(51) Int. Cl.$^4$: **C 07 D 311/82,**
G 01 N 33/533, C 09 K 11/07,
G 01 N 33/52, G 01 N 33/58

(54) **Novel ether substituted fluorescein compounds as fluorescers and quenchers.**

(43) Date of publication of application:
**05.05.82 Bulletin 82/18**

(45) Publication of the grant of the patent:
**22.01.86 Bulletin 86/04**

(84) Designated Contracting States:
**DE FR GB IT SE**

(56) References cited:
**EP-A-0 015 695**
**US-A-3 996 345**
**US-A-3 998 943**
**US-A-4 199 559**

(73) Proprietor: **SYVA COMPANY**
**3181 Porter Drive**
**Palo Alto, CA 94304 (US)**

(72) Inventor: **Khanna, Pyare Lal**
**2235 California Street 179**
**Mountain View California 94040 (US)**
Inventor: **Ullman, Edwin F.**
**135 Selby Lane**
**Atherton California 94025 (US)**

(74) Representative: **Dipl.-Phys.Dr. Manitz Dipl.-Ing. Finsterwald Dipl.-Ing. Grämkow Dipl.-Chem.Dr. Heyn Dipl.-Phys.Rotermund B.Sc. Morgan Robert-Koch-Strasse 1**
**D-8000 München 22 (DE)**

**EP 0 050 684 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Fluorescing compounds find wide application, because of their ability to emit light upon excitation with energy within certain energy ranges. By virtue of this ability, fluorescers have found employment in advertising, novelty items, and as labels in chemical or biological processes, e.g. assays. That is, various compounds can be conjugated to a fluorescing compound, the conjugate subjected to some type of partitioning, and the fate of the conjugate determined by irradiating the sample with light and detecting the zone in which the conjugate exists.

This technique can be employed in immunoassays, involving specific binding pairs, such as antigens and antibodies. By conjugating a fluorescer to one of the members of the specific binding pair and employing various protocols, one can provide for partitioning of the fluorescer conjugate between a solid phase and a liquid phase in relation to the amount of antigen in an unknown sample. By measuring the fluorescence of either of the phases, one can then relate the level of fluorescence observed to a concentration of the antigen in the sample.

Alternatively, one can avoid partitioning of the fluorescent label by providing for a mechanism which varies the fluorescence of the label, depending upon the label environment in a liquid medium. For example, in addition to labeling one of the members of the specific binding pair with the fluorescer, one may label the other member with a quencher, that is, a molecule which is able to absorb the excitation energy of the fluorescer molecule, preventing the emission of a photon. The quenching then will occur only when the two members of the specific binding pair are associated, so that fluorescer and quencher have the required spatial proximity for quenching.

In preparing fluorescers, there are many desiderata. For a fluorescer, one desires a high extinction co-efficient, a high quantum efficiency, preferably approaching or equal to one, chemical stability, a large Stokes shift, and, where the flurescence is to be affected by another agent, an efficient response to such reagent. Furthermore, where the flurescer is to be used in the presence of serum or other composition, which is in itself fluorescent, it is desirable that the fluorescer absorb energy in a substantially different range from that absorbed by the other compounds in the medium. In the case of serum, it is desirable to have fluorescers which absorb light substantially in excess of 450nm, preferably in excess of 500nm.

For quencher molecules, it is desirable that the quencher efficiently quench the fluorescer molecule, that is, that there be substantial overlap between the wavelength range of emission of the fluorescer and the wavelength range of absorption by the quencher. In addition, the quencher should be chemically stable, preferably non-fluorescent, and provide a fluorescer-quencher pair with a high quenching efficiency.

In addition, any compounds of interest should be susceptible to reasonable modes of synthesis to provide the desired product in substantially pure form.

U.S. Patent No. 3,998,943 discloses an immunoassay involving a ligand-fluorescer conjugate employing steric inhibition of simultaneous binding of antibody for ligand and antibody for fluorescer, where the antibody for fluorescer substantially quenches the fluorescence. U.S. Patent No. 3,996,345 describes an immunoassay involving fluorescer-quencher pairs, where a fluorescer is bonded to one member of a specific binding pair and a quencher bonded to the same or different member of a specific binding pair. The assay is dependent upon the degree to which the quencher and fluorescer are brought within quenching proximity based on the amount of analyte in the medium.

EP—A—0 015 695 discloses conjugates of heavy atoms containing analytes or their analogs and fluorescent molecules. Said conjugates are covalently bonded to macromolecular supports to minimize the interference of fluorescence during assays, due to non-specific binding of serum proteins to the conjugate.

There is an extensive list of compounds involving derivatives of fluorescers. Known compounds include 4',5' - dihydroxyflurescein and 4',5' - dihydroxy - 2',7' - dibromofluorescein (C.A. *61*, 7407d). Isothiocyanate derivatives of fluorescein are commercially available, while isocyanate derivatives are described in C.A. *59*, 563b and sulfonic derivatives are described in C.A. *58*, 9012a.

The subject invention concerns chromogenic di(chalcogen ether) symmetrically substituted fluorescein compounds capable of accepting or donating electronic energy, which find particular use when conjugated to other compounds, particularly polypeptides, or soluble or insoluble supports for use as reagents in immunoassays.

The present invention provides a compound of the formula

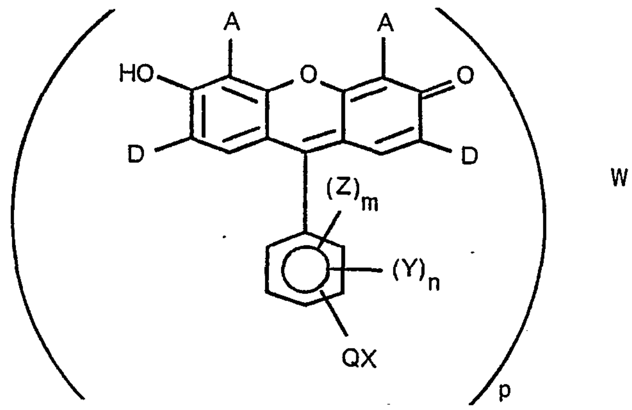

wherein:

the As are the same or different;

the Ds are the same or different;

either the As or the Ds are ethers of the formula —JMX, wherein J is oxygen or sulfur; when other than ethers, they are hydrogen, fluorine, chlorine, bromine, or iodine;

M is a divalent hydrocarbon group of from 1 to 8 carbon atoms;

W is a ligand, receptor, support, or may be taken together with X to form a reactive group for linking;

one of the Xs is a linking group to W; the other X being hydrogen or a group R with the proviso that when X is bonded to a ring carbon atom, X can be halo;

Q is a bond or linking chain;

Y is fluorine, chlorine, bromine or iodine;

Z is an acidic anionic group;

m is an integer of from 0 to 3;

n is an integer of from 0 to 4, wherein m plus n is not greater than 4;

p is at least 1 and up to the molecular weight of W divided by 500;

the group R, when used herein, means the following groups: carboxylic acid, ester, amide, sulfonamide and sulfonic acid.

The fluorescers have large extinction coefficients, high quantum yields, have absorption maxima above 500nm, have Stokes shifts, normally in excess of 10nm and are stable by themselves and when bonded to other compounds. The quenchers have absorption maxima above 500nm, have little or no observable fluorescence and efficiently quench a broad spectrum of fluorescent compounds.

The molecules will have not more than about 45 carbon atoms, usually not more than about 35 carbon atoms. There will be at least 5 oxygen and sulfur atoms, of which at least 3 will be oxygen. In addition to the oxygen and sulfur atoms, there may be from 0 to 8, usually from 0 to 6 heteroatoms, such as nitrogen, fluorine, chlorine, bromine and iodine, or other heteroatoms which may be present to provide specific effects. There will usually be at least one anionic group, normally carboxylate or sulfonate, and one linking group, inter alia the group R as hereinbefore defined, including isothiocyanate and isocyanate; sulfonamide, mercapto, and amino, which may or may not be bonded directly to a ring carbon atom. Suitably, the linking group will be on the phenyl, substituted at the nine position of the xanthene, although linking groups may also be present as substituents on the ether group. These compounds are conjugated to haptens and antigens to provide conjugates which are capable of fluorescing or of quenching a fluorescer when the quencher is in close spatial proximity to the fluorescer.

The subject compositions have absorption maxima above 500nm, usually above 510nm, with relatively narrow bands, usually at least 50% of the area of the longest wavelength absorption being over a wavelength range of about 50 nm. The fluorescing compounds are characterized by having good chemical stability, large Stokes shifts and extinction coefficients in excess of 65,000, usually in excess of 75,000. The Stokes shifts will be at least 10nm, and preferably at least about 20nm. The quenching compounds, will fluoresce with a quantum efficiency less than 10%, preferably less than 5%, in 0.05 molar phosphate when irradiated with light at the absorption maximum.

The compounds of the subject invention provide novel compounds having important spectroscopic and physical properties. The compounds have absorption maxima above 500nm. By choosing the positions for the oxy substituents one can provide highly fluorescent compounds or compounds that are substantially non-fluorescent and can be used as quenchers. Compounds with ether substituents at the 2,7-positions (flurescein numbering) provide fluorescent compounds with high quantum efficiencies. Compounds with ether substituents at the 4,5-positions provide compounds with substantially no fluorescence, while absorbing at long wavelengths so as to act as efficient quenchers.

With the quencher molecules, the 4,5-diether-9-phenyl-6-hydroxy-3H-xanthen-3-ones, the presence or absence of a substituent at the 2 and 7 positions does not affect the quenching, but can be used to modify the absorption characteristics of the molecule. Therefore, when the As are ethers, the Ds may be hydrogen,

fluorine, chlorine, bromine or iodine. The choice of substitution will be governed by the resulting absorption maximum, synthetic convenience and the effect on the physical and chemical properties of the molecule, such as water solubility, chemical reactivity or oxidation sensitivity.

With the fluorescer molecule, the substituents at the 4,5-positions may be hydrogen, fluorine, chlorine, bromine or iodine, particularly chlorine and iodine.

In the formula previously stated M is a divalent hydrocarbon group, normally saturated aliphatic, of from 1 to 8, usually 1 to 6 and preferably 1 to 3 carbon atoms, usually straight chain;

When W is taken together with X to provide a reactive group for linking, XW can be the group R, e.g. carboxylic acid, carboxylic acid ester, e.g. lower alkyl (1—3 carbon atoms) or active ester capable of forming amide bonds in an aqueous medium, e.g. N-oxy succinimide and p-nitrophenyl, isocyanate, isothiocyanate, imidate lower alkyl ester; activated olefin, e.g. maleimido; mercaptan (—SH); formyl (—CHO); sulfonyl chloride; amino; active halo e.g. haloacetyl or halotriazine, with the proviso that XW is the group R when bonded to M;

When W is not taken together with X, one of the Xs is a linking group bonded to W and depending upon the particular active group will be carbonyl or sulfonyl having one valence to carbon; carbamyl, thiocarbamyl; substituted ethylene from activated olefin; thio; methylene (from formyl by reductive amination); amido nitrogen or *sec*-amino; sulfonyl; or oxo-methyl from active halo; when X is a linking group bonded to M, X is carbonyl or sulfonyl; when not a linking group, X is hydrogen or the group R, i.e. carboxylic acid, ester or amide, sulfonamide, sulfonic acid or, particularly when bonded to a ring carbon atom, halo;

p is one when W is taken together with X and is otherwise at least 1 and up to the molecular weight of W divided by 500, generally p ranges from about 1 to 200, usually 1 to 100;

When W is not taken together with X, W is a ligand, including receptors, of at least 125 molecular weight, being haptenic or antigenic, generally being from 125 to 2000 molecular weight when haptenic and from 5000 to $1\times10^7$ when antigenic, although combinations of antigens and other materials may have a much higher composite molecular weight; the ligand will be joined to X, normally through amino, hydroxy, mercapto or active ethylene, to form amido, amidine, thioamide, ether, or thioether, although other linkages may be employed, or W is a soluble or insoluble support which may be a polysaccharide, naturally occurring or synthetic, modified or unmodified, a naturally occurring or synthetic polymer, glass, inorganic solids, or liposomes;

Q is a bond or linking chain, usually aliphatic, aromatic, heterocyclic, or combination thereof, generally aliphatically saturated, where the linking chain will usually have from 1 to 16, more usually 1 to 12, preferably 1 to 8 atoms in the chain, which are carbon, nitrogen, oxygen and sulfur, wherein the nitrogen is amido or bonded solely to carbon and hydrogen, e.g. *tert*-amino, oxygen is oxy, and sulfur is thioether, with the chalcogen bonded solely to carbon and heteroatoms being separated by at least two carbon atoms when bonded to saturated carbon atoms; the total number of carbon atoms being generally 1 to 20 usually 1 to 12 and the total number of heteroatoms being 0 to 10, usually 0 to 8; oxygen may be present as carbonyl or oxy, there being from 0 to 9, usually 0 to 4 heteroatoms; when X is not a reactive group from linking or a linking group, Q will normally be a bond;

Y is fluorine, chlorine, bromine or iodine, particularly chlorine;

n is an integer of from 0 to 4 wherein m plus n is not greater than 4;

Z is an acidic anionic group, such as carboxylic acid or sulfonic acid; and

m is an integer of from 0 to 3, usually 1 to 3.

The subject compounds have many desirable properties. The products have significant water solubility which allow them to be conjugated to a wide variety of polypeptides, without significantly adversely affecting the water solubility of the polypeptide, nor having the polypeptide adversely affect the spectroscopic properties of the subject compounds.

As for the spectroscopic properties of the compounds, the compounds absorb at relatively long wavelengths, generally in excess of 500nm, more usually in excess of 510nm. Thus, naturally occurring fluorescence which may be encountered when working with physiological fluids is substantially avoided by employing exciting light at a wavelength range which does not significantly excite the naturally occurring fluorescers. In addition, the compounds have relatively sharp absorption peaks, and the fluorescers relatively sharp emission peaks. Because of this, efficient overlap can be obtained between fluorescers and quenchers which allow for efficient quenching up to distances of about 70 Å. The fluorescing compounds also have large Stokes shifts, so that the absorption band and emission band peaks are separated by at least 10nm, frequently by at least 15nm. The large Stokes shifts minimize background interference with the observed fluorescence.

The quenchers have little or no fluorescence, so they do not contribute to background interference with the observed signal. By providing for fluorescer-quencher couples, where the absorption band of the quencher substantially overlaps the emission bands of the fluorescer, efficient systems are provided for performing immunoassays, which rely on quenching of fluorescence, when a quencher is brought into close proximity to the fluorescer due to binding of immunologically related materials.

In describing the subject invention, the simple monomeric spectroscopically active compounds used for conjugation will be considered first, followed by consideration of the various conjugates. The

compounds are chemically stable, even at basic pHs, so that they maintain their spectroscopic properties during use.

The compounds employed for conjugation to other compounds will be characterized by having a reactive group for linking which forms a stable covalent bond with another compound, usually an amide bond or thioether bond. For the most part, the reactive group for linking will involve the group R as hereinbefore defined and may be bonded directly to a ring carbon atom of the phenyl group of the fluorescein, bonded through a linking group, or bonded directly or through a linking group to the oxy- or thioether group. Various reactive groups may be employed which are compatible with the other groups in the molecule. These reactive groups for linking include carboxylic acid, which may be activated with carbodiimide or activating alcohols to provide active ester groups, isocyanates, isothiocyanates, imidates or the like which groups react with amino groups to form amides, thioamides or amidines. Alternatively, one can have amino groups, which can be combined with carboxylic acids or derivatives to provide amide links. Finally, one can employ mercapto groups, which can be combined with ethylenic groups, particularly activated ethylenic groups, such as maleic acid derivatives, or vice versa, to provide thioethers.

Preferred compounds of the formula previously stated are those wherein:

the As are the same and

the two Ds are the same;

either the As or the Ds are oxyethers of the formula $—OR^bX^b$; when other than $—OR^bX^b$, they are hydrogen, fluorine, chlorine, bromine or iodine, wherein $R^b$ is a saturated aliphatic hydrocarbon of from 1 to 3 carbon atoms, preferably straight chain and of from 1 to 2 carbon atoms, i.e. methylene or ethylene;

one of X and $X^b$ is a linking group to W, wherein said linking group includes a carbonyl or sulfonyl group;

when other than a linking group, $X^b$ is hydrogen or carboxyl and X is hydrogen, carboxyl, fluorine, chlorine, bromine or iodine;

Q is a bond or linking chain, being a bond when X is other than a linking group; when Q is a linking chain, it has a chain of from 1 to 16 atoms which are carbon, sulfur, oxygen and nitrogen, wherein heteroatoms are spaced apart by at least two carbon atoms when bonded to saturated carbon atoms;

Z is a carboxylic acid or sulfonic acid group;

Y is fluorine, chlorine, bromine or iodine;

m is an integer from 1 to 2;

n is an integer from 0 to 4, wherein m plus n is not greater than 4;

W, when not taken together with X or $X^b$ to form a reactive group for linking, is a ligand, receptor or support;

p is 1 when one of X or $X^b$ is taken together with W, and is otherwise at least 1 and up to the molecular weight of W divided by 500;

When W is taken together with one of the X and $X^b$, usually X, to form a reactive group for linking, this group may have the same definition as —XW, but will usually be a mixed anhydride, e.g. with butyl chloroformate, carboxylic acid, activated ester, isocyanate or isothiocyanate, with the proviso that $X^b$ when taken together with W is a carboxylic acid or derivative thereof;

When W is not taken together with X or $X^b$, one of the X and $X^b$ is a linking group to W, which is carbonyl, forming an amide or ester with W, carbamyl forming a urea with W, or thiocarbamyl forming a thiourea with W;

When Q is a linking chain it has usually a chain of from 1 to 12, usually 2 to 12 atoms in the chain which are carbon, nitrogen, oxygen and sulfur, generally having from 1 to 10, usually 1 to 8 carbon atoms and 0 to 8 heteroatoms which are nitrogen, oxygen and sulfur, wherein oxygen is present bonded solely to carbon e.g. carbonyl or oxy ether, sulfur is analogous to oxygen and nitrogen is amido or bonded solely to carbon e.g. tertiary amino;

When W is not taken together with X or $X^b$, W is a ligand, receptor or support, usually having amino or hydroxyl, particularly amino groups for linking.

The preferred compounds wherein W is taken together with X to which Q is linked are those wherein:

either the As or Ds are alkoxy of from 1 to 3, usually 1 to 2 carbon atoms, when other than alkoxy they are hydrogen, fluorine, chlorine, bromine or iodine;

Y is fluorine, chlorine, bromine or iodine;

Z is carboxy;

m is an integer of from 1 to 2;

n is an integer of from 0 to 4; wherein m plus n is not greater then 4;

W when taken together with X to form a reactive group for linking is the group R as hereinbefore defined and includes acyl halides, mixed anhydrides and, activated ester, as well as isocyanate and isothiocyanate.

Q is a bond or linking chain of from 1 to 12, usually 1 to 10 atoms in the chain which are carbon, nitrogen, oxygen and sulfur, usually carbon and nitrogen, and has from 1 to 12, usually 1 to 10 carbon atoms and 0 to 8, usually 0 to 6 heteroatoms which are nitrogen, oxygen and sulfur, particularly nitrogen and oxygen, wherein nitrogen is present as amido or bonded solely to carbon and the chalcogens (oxygen and sulfur) are bonded solely to carbon, doubly bonded (oxo and thiono) or singly bonded (oxy or thio); as

a linking chain Q can be alkylene of from 1 to 8, usually 1 to 4 carbon atoms, glycyl or polyglycyl of from 1 to 4 glycyl units where the final carboxy is W, or the like;

The following is a list of compounds in this invention which find use for conjugating.

TABLE I

The compounds are substituted 9-phenyl-6-hydroxy-3H-xanthen-3-ones. The numbering in this list is based on the former naming.

2,7-dimethoxy-9-(2′,4′-dicarboxyphenyl)-6-hydroxy-3H-xanthen-3-one
2,7-diethoxy-9-(2′,3′,4′-tricarboxyphenyl)-6-hydroxy-3H-xanthen-3-one
2,7-diethoxy-9-(2′,4′,5′-tricarboxy-3′,6′-dichlorophenyl)-6-hydroxy-3H-xanthen-3-one
2,7-dipropoxy-4,5-dichloro-9-(2′,4′-dicarboxy-6-sulfonatophenyl)-6-hydroxy-3H-xanthen-3-one
2,7-dimethoxy-4,5-dibromo-9-(2′,4′,5′-tricarboxy-phenyl)-6-hydroxy-3H-xanthen-3-one
2,7-dimethoxy-4,5-dichloro-9-(2′,4′,5′-tricarboxyphenyl)-6-hydroxy-3H-xanthen-3-one
2,7-dimethoxy-9-(3′,4′-dicarboxyphenyl)-6-hydroxy-3H-xanthen-3-one
2,7-dimethoxy-9-(3′,4′-dicarboxy-2′,5′,6′-trichlorophenyl)-6-hydroxy-3H-xanthen-3-one
2,7-(2″-carboxyethoxy)-9-(2′-carboxyphenyl)-6-hydroxy-3H-xanthen-3-one
2,7-diethoxy-4,5-dibromo-9-(2′,4′,5′-tricarboxyphenyl)-6-hydroxy-3H-xanthen-3-one
2,7-dimethoxy-9-[2′-carboxy-4′-fluoro-5′-(N-carboxymethyl carboxamide)phenyl]-6-hydroxy-3H-xanthen-3-one
2,7-dimethoxy-9-(2′,4′-dicarboxy-5′-aminophenyl)-6-hydroxy-3H-xanthen-3-one
2,7-dimethoxy-9-(2′-carboxy-4′-isothiocyanatophenyl-6-hydroxy-3H-xanthen-3-one
2,7-diethoxy-9-(2′-carboxy-4′-mercaptophenyl)-6-hydroxy-3H-xanthen-3-one
2,7-dimethoxy-4,5-dibromo-9-(2′-carboxy-4′-mercaptomethylphenyl)-6-hydroxy-3H-xanthen-3-one
2,7-dimethoxy-4,5-dichloro-9-(2′-carboxy-4′-cyanophenyl)-6-hydroxy-3H-xanthen-3-one
4,5-dimethoxy-9-(2′,4′,5′-tricarboxy phenyl)-6-hydroxy-3H-xanthen-3-one
4,5-dimethoxy-9-(2′,5′-dicarboxy phenyl)-6-hydroxy-3H-xanthen-3-one
4,5-dimethoxy-2,7-diiodo-9-(2′,4′,5′-tricarboxy phenyl)-6-hydroxy-3H-xanthen-3-one
4,5-dimethoxy-2,7-dichloro-9-(2′,4′,5′-tricarboxy phenyl)-6-hydroxy-3H-xanthen-3-one
4,5-dimethoxy-2,7-dichloro-9-(2′,4′,5′-tricarboxy-3′,6′-dichlorophenyl)-6-hydroxy-3H-xanthen-3-one
4,5-di(2″-carboxyethoxy)-2,7-diiodo-9-(2′-carboxy-4′-sulfonatophenyl)-6-hydroxy-3H-xanthen-3-one
4,5-dipropoxy-2,7-dibromo-9-(2′,4′-dicarboxy-3′,5′,6′-trichlorophenyl)-6-hydroxy-3H-xanthen-3-one
4,5-diethoxy-2,7-dichloro-9-(2′-carboxy-4′-amino-5′-sulfonatophenyl)-6-hydroxy-3H-xanthen-3-one
4,5-dimethoxy-2,7-diiodo-9-(2′-carboxy-4′-isothiocyanatophenyl)-6-hydroxy-3H-xanthen-3-one
4,5-diethoxy-2,7-diiodo-9-[2′,4′-dicarboxy-5′-(N-carboxymethyl formamide)phenyl]-6-hydroxy-3H-xanthen-3-one
4,5-dimethoxy-2,7-dichloro-9-(2′,4′-dicarboxy-3′,5′,6′-trichlorophenyl)-6-hydroxy-3H-xanthen-3-one
4,5-diethoxy-2,7-dichloro-9-(4′,5′-dicarboxy-2′,3′,6′-trichlorophenyl)-6-hydroxy-3H-xanthen-3-one
4,5-dimethoxy-2,7-diiodo-9-(2′-carboxy-4′-mercaptomethylphenyl)-6-hydroxy-3H-xanthen-3-one

The above list is intended to merely be illustrative and not exhaustive of the compounds included within the scope of the subject invention.

The compounds of this invention having a reactive group for linking may be conjugated to ligands or supports having complementary heteroatom groups capable of conjugating. The following table indicates illustrative linking groups of ligands, groups for linking the compounds of this invention with the ligands and the resulting linking unit.

| Spectroscopic compound reactive groups for linking* | Ligand reactive groups for linking | Resulting linking group |
|---|---|---|
| —CO—OH, —$OT^1$, —$OCO_2T$ | $NH_2$ | —CO—NH— |
| —NCO | $NH_2$ | —NHCONH— |
| —NCS | $NH_2$ | —NHCSNH— |
| 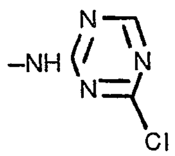 | $NH_2$ | 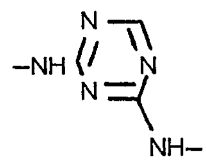 |
| —C=C—CO— | SH | S—C—C—CO— |
| —CHO | $NH_2$ | —$CH_2$NH— |
| —$SO_2$Cl | $NH_2$ | —$SO_2$NH— |
| —$COCH_2$ halo | SH or OH | —$COCH_2$—S— or —O— |

*T is alkyl; $T^1$ is alkyl or electronegative ester activating group.

In most cases, the linking group of the ligand and the linking group of the spectroscopic compound may be switched.

Suitably the ligand conjugates of the formula previously stated are those wherein:

either the As or the Ds are alkoxy of from 1 to 3 carbon, when other than alkoxy, they are hydrogen, fluorine, chlorine, bromine or iodine;

Z is carboxyl;

m is an integer of from 1 to 2;

Q is a bond or linking chain of from 1 to 12 atoms in the chain, which are carbon, nitrogen and oxygen;

X to which Q is linked is carbonyl or sulfonyl;

W is s ligand of at least 125 molecular weight, a receptor or a support;

p is at least 1 and up to the molecular weight of W divided by 500.

With haptenic ligands of from about 125 to 2000 molecular weight, p will usually be 1; with antigenic ligands of greater than 2000 molecular weight, usually greater than 5000 molecular weight, p will generally be on the average from about 1 to 200, usually 1 to 100 and more usually 2 to 50; and

As a ligand W will be haptenic or antigenic; haptenic ligands will generally be from about 125 to 2000 molecular weight and will have at least one polar group, which may or may not be the site for linking; antigenic ligands will be polyepitopic, will usually be poly(amino acids), nucleic acids, polysaccharides and combinations thereof, generally of at least 2000, usually at least 5000 molecular weight and may be 10 million or more molecular weight; supports may be of indeterminate molecular weight, usually at least 10,000, more usually at least 20,000 molecular weight and may be 10 million or more, with soluble supports generally under 10 million molecular weight; the supports may be naturally occurring or synthetic, may be particulate or have a shaped form; swellable or non-swellable by aqueous media; the support may be cross-linked or non-cross-linked, may be a single substance or a mixture of substances; naturally occurring supports may include polysaccharides, nucleic acids, poly(amino acids) e.g. polypeptides and proteins, rubbers, lignin, vesicles, combinations thereof; synthetic supports may include addition and condensation polymers e.g. polystyrene, polyacrylics, vinyl compounds, polyesters, polyethers and polyamides; charcoal, metal chalcogenides, glass or liposomes.

A preferred group of ligand conjugates of the formula previously stated is that wherein:

for quencher molecules, the As are alkoxy of from 1 to 2 carbon atoms and the Ds are hydrogen, fluorine, chlorine, bromine or iodine, particularly chlorine, bromine or iodine;

for fluorescer molecules, the Ds are alkoxy of from 1 to 2 carbon atoms and the As are hydrogen, fluorine, chlorine, bromine or iodine, particularly chlorine or bromine;

Z is carboxyl;

Y is fluorine, chlorine, bromine, iodine, particularly chlorine;

Q is a bond or linking chain of from 1 to 9, usually 2 to 9 atoms in the chain, which are carbon and nitrogen, particularly amido nitrogen, wherein the linking chain is composed solely of carbon, oxygen, nitrogen and hydrogen, wherein oxygen is bonded solely to carbon as carbonyl; useful linking chains include alkylene of from 1 to 6, usually 2 to 4 carbon atoms, mono- or polyamidomethylene ($-CONHCH_2-$) aminomono- or amino polyamidomethylene ($-NH(CONHCH_2)_x$), or aminothiono mono- or poly(amino-methylenecarbonyl) amino-methylene ($-NHCS(NHCH_3CO)_x$, $NHCH_2-$), wherein x is 1 to 3;

X to which Q is linked is the group R, as hereinbefore defined, carbamyl or thiocarbamyl, bonded to amino of W to form an amide bond;

W is a receptor or ligand, usually haptenic of from about 125 to 1000 molecular weight of antigen of from about 2000 to 10 million, usually 5000 to 2,000,000, more usually 5000 to 1,000,000 molecular weight; W is particularly a poly(amino acid) or polysaccharide antigen and may be any hapten;

p is at least 1 and up to the molecular weight of W divided by 500, generally in the range of about 1 to 200, more usually in the range of about 2 to 150, and frequently in the range of about 1 to 75. For ligands below 500,000 molecular weight p will generally be in the range of about 2 to 50;

m is an integer of from 1 to 2;

n is an integer of from 0 to 3, with m plus n not greater than 4.

Particular ligand conjugates are exemplified by the ligand conjugates claimed in Claims 14, 16 and 17.

In some instances it may be desirable to have the conjugate of the ligand and subject fluorescer bound, either covalently or non-covalently to a support. The binding of the conjugate to a support may be by means of a covalent bond to a group either on the ligand or subject fluorescer or by non-covalent binding between the ligand, normally polyepitopic, and a receptor, usually an antibody, which in turn may be covalently or non-covalently bound to the support. In these cases in the formula previously stated W is a ligand or receptor which is bound to the support. Especially W may be a ligand which is bound to a macromolecular support of at least 10,000 molecular weight.

For use in immunoassays or in other diagnostic situations, the spectroscopically active compounds of this invention will be conjugated to a compound of interest, including a receptor for an analyte or a ligand. (By receptor is intended any compound which is capable of recognizing, and binding to, a particular structural feature on the ligand). The analyte will normally be haptenic or antigenic. Where these compounds do not have available reactive groups for linking, they will be modified to introduce such a group, while still retaining the receptor recognition properties in the resulting product. These compounds which are analogues of the analyte, which analyte may also be referred to as a ligand, will be referred to as ligand analogues.

As indicated previously, the compounds of this invention may be conjugated to compounds which may be measured by known immunoassay techniques. The resulting conjugates are reagents which compete in an assay medium with the compound of interest or analyte in a sample. Therefore, the conjugate retains a sufficient proportion of the structure of the compound of interest to be able to compete with the compound of interest for receptor, usually an antibody.

The analytes or their analogues, receptors or ligands, which are conjugated to the spectroscopically active compounds of this invention are characterized by being monoepitopic or polyepitopic.

Analyte

The ligand analytes of this invention are characterized by being monoepitopic or polyepitopic. The polyepitopic ligand analytes will normally be poly(amino acids) i.e. polypeptides and proteins, polysaccharides, nucleic acids, and combinations thereof. Such combinations of assemblages include bacteria, viruses, chromosomes, genes, mitochondria, nuclei and cell membranes.

Suitably the polyepitopic ligand analytes employed in the subject invention will have a molecular weight of at least 5,000, more usually at least 10,000. In the poly(amino acid) category, the poly(amino acids) of interest will generally be from 5,000 to 5,000,000 molecular weight, more usually from about 20,000 to 1,000,000 molecular weight; among the hormones of interest, the molecular weights will usually range from 5,000 to 60,000 molecular weight.

The wide variety of proteins may be considered as to the family of proteins having similar structural features, proteins having particular biological functions, proteins related to specific microorganisms, particularly disease causing microorganisms, etc.

The following are classes of proteins related by structure:

protamines
histones
albumins
globulins
scleroproteins
phosphoproteins
mucoproteins
chromoproteins
lipoproteins
nucleoproteins

glycoproteins
proteoglycans
unclassified proteins, e.g. somatotropin, prolactin, insulin, pepsin

A number of proteins found in the human plasma are important clinically and include:

Prealbumin
Albumin
$\alpha_1$-Lipoprotein
$\alpha_1$-Acid glycoprotein
$\alpha_1$-Antitrypsin
$\alpha_1$-Glycoprotein
Transcortin
4.6S-Postalbumin
Tryptophan-poor $\alpha_1$-glycoprotein
$\alpha_1\chi$-Glycoprotein
Thyroxin-binding globulin
Inter-$\alpha$-trypsin-inhibitor
Gc-globulin
(Gc 1-1)
(Gc 2-1)
(Gc 2-2)
Haptoglobin
(Hp 1-1)
(Hp 2-1)
(Hp 2-2)
Ceruloplasmin
Cholinesterase
$\alpha_2$-Lipoprotein(s)
Myoglobin
C-Reactive Protein
$\alpha_2$-Macroglobulin
$\alpha_2$-HS-glycoprotein
Zn-$\alpha_2$-glycoprotein
$\alpha_2$-Neuramino-glycoprotein
Erythropoietin
$\beta$-lipoprotein
Transferrin
Hemopexin
Fibrinogen
Plasminogen
$\beta_2$-glycoprotein I
$\beta_2$-glycoprotein II
Immunoglobulin G (IgG) or $\gamma$G-globulin
Mol. formula: $\gamma_2\kappa_2$ or $\gamma_2\lambda_2$
Immunoglobulin A (IgA) or $\gamma$A-globulin
Mol. formula: $(\alpha_2\kappa_2)^n$ or $(\alpha_2\lambda_2)^n$
Immunoglobulin M (IgM) or $\gamma$M-globulin
Mol. formula: $(\mu_2\kappa_2)^5$ or $(\mu_2\lambda^2)^5$
Immunoglobulin D(IgD) or $\gamma$D-Globulin ($\gamma$D)
Mol. formula $(\delta_2\kappa_2)$ or $(\delta_2\lambda_2)$
Immunoglobulin E (IgE) or $\gamma$E-Globulin ($\gamma$E)
Mol. formula: $(\varepsilon_2\kappa_2)$ or $(\varepsilon_2\lambda_2)$
Free $\kappa$ and $\lambda$ light chains

Complement factors:
C'1
C'1q
C'1r
C'1s
C'2
C'3
$\beta_1A$
$\alpha_2D$
C'4
C'5
C'6
C'7
C'8
C'9

Important blood clotting factors include:

Blood clotting factors

| International designation | Name |
|---|---|
| I | Fibrinogen |
| II | Prothrombin |
| IIa | Thrombin |
| III | Tissue thromboplastin |
| V and VI | Proaccelerin, accelerator globulin |
| VII | Proconvertin |
| VIII | Antihemophilic globulin (AHG) |
| IX | Christmas factor, plasma thromboplastin component (PTC) |
| X | Stuart-Prower factor, autoprothrombin III |
| XI | Plasma thromboplastin antecedent (PTA) |
| XII | Hagemann factor |
| XIII | Fibrin-stabilizing factor |

Important protein hormones include:

Peptide and protein hormones
Parathyroid hormone
(parathromone)
Thyrocalcitonin
Insulin
Clucagon
Relaxin
Erythropoietin
Melanotropin
(melanocyte-stimulating hormone; intermedin)
Somatotropin
(growth hormone)
Corticotropin
(adrenocorticotropic hormone)
Thyrotropin
Follicle-stimulating hormone

Luteinizing hormone
(interstitial cell-stimulating hormone)
Luteomammotropic hormone
(luteotropin, prolactin)
Gonadotropin
(chorionic gonadotropin)

Tissue Hormones
Secretin
Gastrin
Angiotensin I and II
Bradykinin
Human placental lactogen

Peptide hormones from the Neurohypophysis
Oxytocin
Vasopressin
Releasing factors (RF)
CRF, LRF, TRF, Somatotropin-RF,
GRF, FSH-RF, PIF, MIF

Other polymeric materials of interest are mucopolysaccharides and polysaccharides. Illustrative antigenic polysaccharides derived from microorganisms are as follows:

| Species of microorganisms | Hemosensitin found in |
|---|---|
| Streptococcus pyogenes | Polysaccharide |
| Diplococcus pneumoniae | Polysaccharide |
| Neisseria meningitidis | Polysaccharide |
| Neisseria gonorrheae | Polysaccharide |
| Corynebacterium diphtheriae | Polysaccharide |
| Actinobacillus mallei; Actinobacillus whitemori | Crude extract |
| Francisella tularensis | Lipopolysaccharide Polysaccharide |
| Pasteurella pestis | |
| Pasteurella pestis | Polysaccharide |
| Pasteurella multocida | Capsular antigen |
| Brucella abortus | Crude extract |
| Haemophilus influenzae | Polysaccharide |
| Haemophilus pertussis | Crude |
| Treponema reiteri | Polysaccharide |
| Veillonella | Lipopolysaccharide |
| Erysipelothrix | Polysaccharide |
| Listeria monocytogens | Polysaccharide |
| Chromobacterium | Lipopolysaccharide |
| Mycobacterium tuberculosis | Saline extract of 90% phenol extracted mycobacteria and polysaccharide fraction of cells and tuberculin |
| Klebsiella aerogenes | Polysaccharide |
| Klebsiella cloacae | Polysaccharide |
| Salmonella typhosa | Lipopolysaccharide, Polysaccharide |
| Salmonella typhi-murium; Salmonella derby Salmonella pullorum | Polysaccharide |
| Shigella dysenteriae | Polysaccharide |
| Shigella flexneri | |
| Shigella sonnei | Crude, Polysaccharide |
| Rickettsiae | Crude extract |
| Candida albicans | Polysaccharide |
| Entamoeba histolytica | Crude extract |

The microorganisms which are assayed may be intact, lysed, ground or otherwise fragmented, and the resulting composition or portion, e.g. by extraction, assayed. Microorganisms of interest include:

Corynebacteria
- Corynebacterium diphtheriae

Pneumococci
- Diplococcus pneumoniae

Streptococci
- Streptococcus pyogenes
- Streptococcus salivarus

Staphylococci
- Staphylococcus aureus
- Staphylococcus albus

Neisseriae
- Neisseria meningitidis
- Neisseria gonorrheae

Enterobacteriaciae

| Species | Group |
|---|---|
| Escherichia coli<br>Aerobacter aerogenes<br>Klebsiella pneumoniae | The coliform bacteria |
| Salmonella typhosa<br>Salmonella choleraesuis<br>Salmonella typhimrium | The Salmonellae |
| Shigella dysenteriae<br>Shigella schmitzii<br>Shigella arabinotarda<br>Shigella flexneri<br>Shigella boydii<br>Shigella Sonnei | The Shigellae |

Other enteric bacilli

| Species | Group |
|---|---|
| Proteus vulgaris<br>Proteus mirabilis<br>Proteus morgani | Proteus species |

- Pseudomonas aeruginosa
- Alcaligenes faecalis
- Vibrio cholerae

Hemophilus-Bordetella group
- Hemophilus influenzae,
  - H. -ducreyi
  - H. hemophilus
  - H. aegypticus
  - H. parainfluenzae
- Bordetella pertussis

Pasteurellae
- Pasteurella pestis
- Pasteurella tulareusis

Brucellae
- Brucella melitensis
- Brucella abortus
- Brucella suis

Aerobic spore-forming bacilli
- Bacillus anthracis
- Bacillus subtilis
- Bacillus megaterium
- Bacillus cereus

Anaerobic spore-forming bacilli
- Clostridium botulinum
- Clostridium tetani
- Clostridium perfringens.
- Clostridium novyi
- Clostridium septicum
- Clostridium histolyticum
- Clostridium tertium
- Clostridium bifermentans
- Clostridium sporogenes

Mýcobacteria
- Mycobacterium tuberculosis hominis
- Mycobacterium bovis
- Mycobacterium avium
- Mycobacterium leprae
- Mycobacterium paratuberculosis

Actinomycetes (fungus-like bacteria)
- Actinomyces israelii
- Actinomyces bovis
- Actinomyces naeslundii
- Nocardia asteroides
- Nocardia brasiliensis

The Spirochetes
- Treponema pallidum
- Treponema pertenue
- Spirillum minus
- Streptobacillus moniliformis
- Treponema carateum
- Borrelia recurrentis
- Leptospira icterohemorrhagiae
- Leptospira canicola

Mycoplasmas
- Mycoplasma pneumoniae

Other pathogens
- Listeria monocytogenes
- Erysipelothrix rhusiopathiae
- Streptobacillus moniliformis
- Donvania grànulomatis
- Bartonella bacilliformis

Rickettsiae (bacteria-like parasites)
- Rickettsia prowazekii
- Rickettsia mooseri
- Rickettsia rickettsii
- Rickettsia conori
- Rickettsia australis
- Rickettsia sibiricus
- Rickettsia akari
- Rickettsia tsutsugamushi
- Rickettsia burnetti
- Rickettsia quintana

Chlamydia (unclassifiable parasites bacterial/viral)
- Chlamydia agents (naming uncertain)

Fungi

- Cryptococcus neoformans
- Blastomyces dermatidis
- Histoplasma capsulatum
- Coccidioides immitis
- Paracoccidioides brasiliensis
- Candida albicans
- Aspergillus fumigatus
- Mucor corymbifer (Absidia corymbifera)

- Rhizopus oryzae
- Rhizopus arrhizus
- Rhizopus nigricans

} Phycomycetes

- Sporotrichum schenkii
- Fonsecaea pedrosoi
- Fonsecaea compacta
- Fonsecaea dermatidis
- Cladosporium carrionii
- Phialophora verrucosa
- Aspergillus nidulans
- Madurella mycetomi
- Madurella grisea
- Allescheria boydii
- Phialosphora jeanselmei
- Microsporum gypseum
- Trichophyton mentagrophytes
- Keratinomyces ajelloi
- Microsporum canis
- Trichophyton rubrum
- Microsporum andouini

Viruses

Adenoviruses

Herpes viruses

- Herpes simplex
- Varicella (Chicken pox)
- Herpes Zoster (Shingles)
- Virus B
- Cytomegalovirus

Pox viruses

- Variola (smallpox)
- Vaccinia
- Poxvirus bovis
- Paravaccinia
- Molluscum contagiosum

Picornaviruses

- Poliovirus
- Coxsackievirus
- Echoviruses
- Rhinoviruses

Myxoviruses

- Influenza (A, B, and C)
- Parainfluenza (1—4)
- Mumps Virus
- Newcastle Disease Virus
- Measles Virus
- Rinderpest Virus
- Canine Distemper Virus
- Respiratory Syncytial Virus
- Rubella Virus

Arboviruses

- Eastern Equine Eucephalitis Virus
- Western Equine Eucephalitis Virus
- Sindbis Virus
- Chikugunya Virus
- Semliki Forest Virus
- Mayora Virus
- St. Louis Encephalitis Virus
- California Encephalitis Virus
- Colorado Tick Fever Virus
- Yellow Fever Virus
- Dengu Virus

Reoviruses

- Reovirus Types 1—3

Hepatitis

- Hepatitis A Virus
- Hepatitis B Virus

Tumor viruses

- Rauscher Leukemia Virus
- Gross Virus
- Maloney Leukemia Virus

The monoepitopic ligand analytes will generally be from about 100 to 2,000 molecular weight, more usually from 125 to 1,000 molecular weight. The analytes of interest include drugs, metabolites, pesticides and pollutants. Included among drugs of interest are the alkaloids. Among the alkaloids are morphine alkaloids, which includes morphine, codeine, heroin, dextromethorphan, their derivatives and metabolites; cocaine alkaloids, which includes cocain and benzoyl ecgonine, their derivatives and metabolites; ergot alkaloids, which includes the diethylamide of lysergic acid; steroid alkaloids; iminazoyl alkaloids; quinazoline alkaloids, isoquinoline alkaloids; quinoline alkaloids; which includes quinine and quinidine; diterpene alkaloids; their derivatives and metabolites.

The next group of drugs includes steroids, which includes the estrogens, gestogens, androgens, andrenocortical steroids, bile acids, cardiotonic glycosides and aglycones, which includes digoxin and digoxigenin, saponins and sapogenins, their derivatives and metabolites. Also included are the steroid mimetic substances, such as diethylstilbestrol.

The next group of drugs is lactams having from 5 to 6 annular members, which include the barbiturates, e.g. phenobarbital and secobarbital, diphenylhydantonin, primidone, ethosuximide, and their metabolites.

The next group of drugs is aminoalkylbenzenzes, with alkyl of from 2 to 3 carbon atoms, which includes the amphetamines, catecholamines, which includes ephedrine, L-dopa, epinephrine, narceine, papaverine, their metabolites.

The next group of drugs is aminoalkylbenzenes, with alkyl of from 2 to 3 carbon atoms, which includes ephedrine, L-dopa, epinephrine, narceine, papverine, their metabolites and derivatives.

The next group of drugs is benzheterocyclics which include oxazepam, chlorpromazine, tegretol, imipramine, their derivatives and metabolites, the heterocyclic rings being azepines, diazepines and phenothiazines.

The next group of drugs is purines, which includes theophylline, caffeine, their metabolites and derivatives.

The next group of drugs includes those derived from marijuana, which includes cannabinol and tetrahydrocannabinol.

The next group of drugs includes the vitamins such as A, B, e.g. $B_{12}$, C, D, E and K, folic acid, thiamine.

The next group of drugs is prostaglandins, which differ by the degree and sites of hydroxylation and unsaturation.

The next group of drugs is antibiotics, which include penicillin, chloromycetin, actinomycetin, tetracycline, terramycin, their metabolites and derivatives, and aminoglycosides, such as gentamicin, kanamicin, tobramycin, and amikacin.

The next group of drugs is the nucleosides and nucleotides, which include ATP, NAD, FMN, adenosine, guanosine, thymidine, and cytidine with their appropriate sugar and phosphate substituents.

The next group of drugs is miscellaneous individual drugs which include methadone, meprobamate, serotonin, meperidine, amitriptyline, nortriptyline, lidocaine, procaineamide, acetylprocaineamide, propranolol, griseofulvin, valproic acid, butyrophenones, antihistamines, anticholinergic drugs, such as atropine, their metabolites and derivatives.

The next group of compounds is amino acids and small peptides which include polyiodothyronines e.g. thyroxine, and triiodothyronine, oxytocin, ACTH, angiotensin, met- and leu-enkaphalin their metabolites and derivatives.

Metabolites related to diseased states include spermine, galactose, phenylpyruvic acid, and porphyrin Type 1.

Among pesticides of interest are polyhalogenated biphenyls, phosphate esters, thiophosphates, carbamates, polyhalogenated sulfenamides, their metabolites and derivatives.

Receptors are compounds capable of binding to a particular spacial and polar organization of a molecule. For the most part, receptors are antibodies, particularly IgG, IgA, IgE and IgM. The receptors may be analytes or may be employed as reagents in immunoassay. In either event, the receptors can be conjugated to the spectroscopically active compounds of this invention.

For receptor analytes, the molecular weights will generally range from 10,000 to $2\times10^6$, more usually from 10,000 to $10^6$. For immunoglobulins, IgA, IgG, IgE and IgM, the molecular weights will generally vary from about 160,000 to about $10^6$. Enzymes will normally range from about 10,000 to 600,000 in molecular weight. Natural receptors vary widely, generally being at least about 25,000 molecular weight and may be $10^6$ or higher molecular weight, including such materials as avidin, thyroxine binding globulin, thyroxine binding prealbumin, transcortin, etc.

The compounds of the subject invention can be conjugated to a wide variety of ligands, haptens and antigens, and receptors to provide conjugates which are used in various assays, particularly immunoassays. Particular conjugates are of interest, because of the greater interest in the analyte as a drug, for example, the ease of quantitation with the compounds of the subject invention. The following are formulas of conjugates of particular interest.

The first conjugate of interest is a lactamchromophore conjugate of the formula

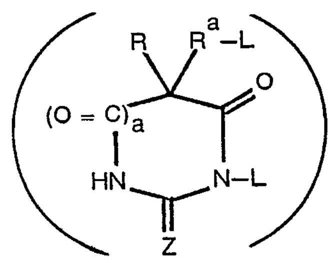

wherein:

a is 1, except when R and $R^a$ are phenyl, when it is 0 or 1,

Z is oxygen or sulfur, normally being oxygen;

R and $R^a$ are hydrocarbon of from 1 to 7 carbon atoms, more usually of from 1 to 6 carbon atoms having from 0 to 1 site of ethylenic unsaturation when other than aromatic e.g. methyl, ethyl, *sec*-butyl, *n*-butyl, α-methylbutyl, isoamyl, hexyl, $\Delta^1$-cyclohexenyl, and phenyl, particularly combinations of alkyl and α-methylbutyl or ethyl and phenyl, when a is 1, and two phenyls, when a is 0;

one of the L's is a bond, or a linking group to Ch, usually ending in oxy, amino, carbonyl or mercapto and having a chain of from 0 to 6, usually 0 to 4 carbon atoms; when other than the linking site, L is hydrogen;

Ch is of the formula:

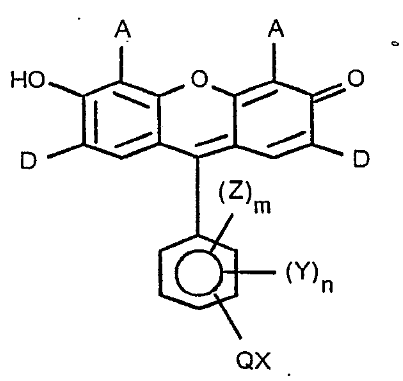

wherein:

A, D, Z, Y, Q, m and n have been defined previously and X to which Q is linked is a linking group to L to form an amide, amidine, urea, thiourea, ester, oxy- or thioether, or *sec*-amino group linking the ligand and chromophore; included in the above formula are all the preceding meanings of narrower scope, where the X to which Q is linked, is the linking group.

The next chromophore ligand conjugates of interest are for theophylline which will have the following formula:

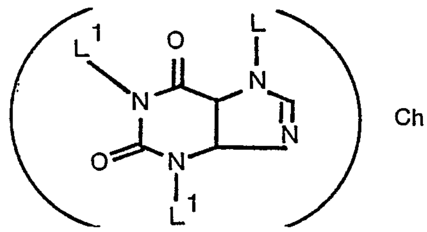

wherein:

one of L and $L^1$ is a bond or a linking group to Ch, usually ending in oxy, amino, carbonyl or mercapto and having a chain of from 0 to 6, usually 0 to 4 carbon atoms; when other than the linking site, L is hydrogen and $L^1$ is methyl; and

Ch has been defined previously.

The next chromophore ligand conjugates of interest are for aminoglycosides, particularly antimicrobial aminoglycosides, which for the most part will have the following formula:

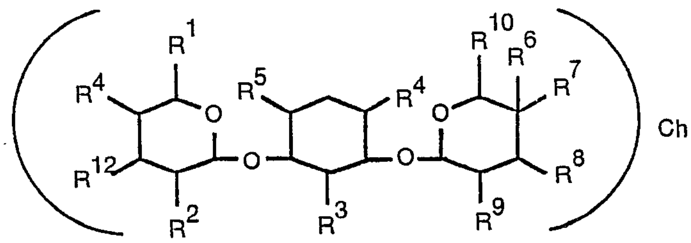

wherein:

L may be bonded to any one of the R groups; otherwise:

$R^2$ is amino ($-NH_2$), except for kanamycin and amikacin, when it is hydroxyl;

$R^3$, $R^7$ and $R^9$ are hydroxyl;

$R^4$ and $R^5$ are amino except for amikacin where $R^4$ is 2-hydroxy-4-aminobutylamido;

$R^6$ is hydrogen or methyl, being methyl when gentamicin and hydrogen when tobramycin, kanamycin or amikacin;

$R^8$ is amino or methylamino, being methylamino when gentamicin and amino otherwise;

$R^{10}$ is hydrogen or hydroxymethyl, being hydrogen for gentamicin and hydroxymethyl otherwise;

$R^{11}$ and $R^{12}$ are hydrogen or hydroxyl, being hydrogen for gentamicin, hydroxyl for kanamycin and amikacin, and $R^{11}$ is hydroxyl and $R^{12}$ is hydrogen for tobramycin; and

L and Ch have been defined previously.

The next chromophore ligand conjugates of interest are for tricyclic antidepressant drugs, which will have the following formula:

0—1 site of ethylenic unsaturation

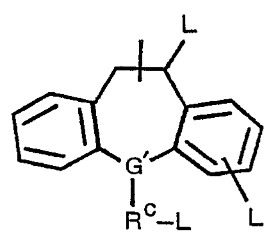

wherein:

one of the L's is a linking group as previously defined, and is otherwise hydrogen;

G' is N—, C= or CH—, the remaining two valences to the adjacent annular carbon atoms;

$R^c$ is a single or double bond joining L to G, or is aminopropyl or -ylidene having from 0 to 2 methyl groups bonded to the nitrogen.

The next chromophore ligand conjugates of interest are for benzodiazepines, which will have the following formula:

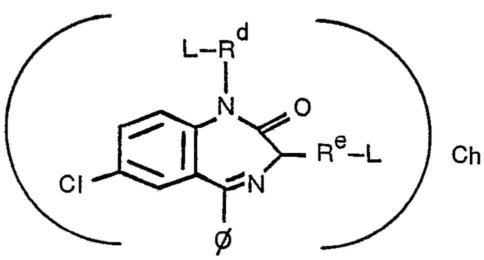

wherein:

L and Ch have been defined previously;

$R^d$ is a bond or methylene when L is hydrogen;

$R^e$ is a bond or oxy; and

Ø is phenyl having from 0 to 1 ortho halogen of atomic number 9 to 17.

The linking group symbolized by L will vary depending upon the nature of R to which L is attached, and the group to which L is bonded. When L is a bond, it will normally join a carbon atom to a heteroatom, particularly nitrogen and oxygen. When L is a linking group, it will normally have a chain of from 1 to 8, usually 1 to 6, more usually 1 to 4, of which 0 to 2, usually 0 to 1 are heteroatoms, which are oxygen, sulfur and nitrogen, the chalcogen being bonded solely to carbon, and the nitrogen being bonded solely to carbon and hydrogen, the chain terminating as methylene or with a heteroatom group, L having a total of 1 to 10, usually 1 to 6 carbon atoms and 0 to 4, usually 1 to 3 heteroatoms, which are oxygen, sulfur and nitrogen.

The subject conjugates can be used in a variety of ways for determining, qualitatively, semi-quantitatively, or quantitatively the presence of a compound of interest in a sample. Where compounds are to be detected in physiological fluids, the fluid may include serum, urine, saliva or lymph. Where the compound of interest is involved in chemical processing or ecological concerns, the sample may involve an aqueous medium, an organic medium, soil, inorganic mixtures, or the like.

The assays will normally involve a change of spectroscopic properties due to a change of environment about the spectroscopically active compound or the bringing together of a fluorescer-quencher pair within sufficient proximity for the quencher to interact with the fluorescer.

In a first assay, steric exclusion is involved, in that receptors or antibodies for the ligand and for the fluorescer are employed, where simultaneous binding of the receptor for the ligand and receptor for the fluorescer is inhibited. Furthermore, when the receptor for the fluorescer (antifluorescer) is bound to the fluorescer, the fluorescence of the fluorescer is substantially diminished. Further reduction if not complete inhibition of fluoresence can be achieved by conjugation of quencher to the antifluorescer. This assay is extensively described in U.S. Patent No. 3,998,943, issued December 21, 1976. The fluorescein which is employed there may be substituted with the fluorescent compounds of the subject invention. The assay is described in columns 3 to 5 of the subject patent.

Generally, the method involves combining the sample suspected of containing the analyte, the conjugate of the ligand and fluorescer, antifluorescer, and receptor for ligand or antiligand, when ligand is the analyte. The materials are combined in an aqueous medium at a pH in the range of 5 to 10, usually in the range of 6 to 9, at a temperature in the range of about 10° to 45°C, and the fluorescence determined either as a rate or equilibrium mode, readings being taken within about 1 second to one hour after all materials have been combined for a rate mode, while for an equilibrium mode, readings may be taken for as long as up to about 24 hours or longer.

In the next immunoassay technique, a fluorescer-quencher pair is employed, where one of the members of the pair is conjugated to a member of a specific binding pair, ligand and antiligand, and the other chromophor member is bound to the same or different member of the specific binding pair. For example, the fluorescer may be bound to antiligand and the quencher may be bound to different molecules of antiligand, so that when the two conjugated antiligands are brought together with antigen, the fluorescer and quencher are brought within quenching distance. Alternatively, one could bond one of the chromophors to the ligand and the other chromophor to the antiligand. This assay is extensively described in U.S. Patent No. 3,996,345. The assay technique is described beginning with column 17 and ending at column 23. The ratios of chromophor to ligand and receptor is described in columns 4 to 6.

The assay is carried out substantially in the same manner as described above, except that in this assay, the fluorescer conjugate and quencher conjugate are added in conjunction with the sample and the fluorescence determined in comparison to an assay medium having a known amount of the analyte.

Other techniques may also be employed with the subject compounds, such as techniques involving heavy atom quenching or other assay techniques where a fluorescent molecule is desired which emits light at a wavelength substantially above the light emitted by fluorescent compounds naturally present in physiological fluids or other samples to be analyzed.

Experimental

The following examples are offered by way of illustration and not by way of limitation.

(All temperatures not otherwise indicated are centigrade. All parts and percents not otherwise indicated are by weight, except for mixtures of liquids, which are by volume. The following abbreviations are employed: THF—tetrahydrofuran; NHS—N-hydroxy succinimide; DMF—N,N-dimethyl formamide DCC—dicyclohexyl carbodiimide; PEG—polyethylene glycol 6000).

Example 1

Preparation of 2-chloro-4-methoxyresorcinol

Into a reaction flask was introduced 2 g of 2-chloro-3-hydroxy-4-methoxybenzaldehyde in 50ml methylene dichloride with an excess of *m*-chloroperbenzoic acid and the reaction refluxed for 2.5 hrs. After removal of the solvent, the solid residue was dissolved in 10ml methanol and stirred with approximately 10

weight percent aqueous sodium hydroxide (10ml) for 1 hr. After acidification to pH1 with dilute HCl, the solution was filtered and the precipitate washed with 10ml of cold water. The combined filtrate and wash was extracted with ether, the ether washed with 5% $NaHCO_3$ (2×10ml) and saturated NaCl solution (1×10ml) and then evaporated to leave a solid which was crystallized from ethyl acetate-hexane mixture to provide the product as white silky needles (1.35g). mp 69—70°.

Example 2
Preparation of 3′,6′-dihydroxy-4′,5′-dichloro-2′,7′-dimethoxyspiro[isobenzofuran-1(3H),9′-[9H]xanthen]-5 or 6-carboxy-3-one

A mixture of the product of Example 1 (200mg) and 100mg of 1,2,4-benzenetricarboxylic anhydride was heated with 15mg zinc chloride at 180—85° for 30 min. The resulting red mixture was dissolved in 5% aqueous sodium hydroxide and precipitated with dilute HCl to pH1. The precipitate was then chromatographed using $CH_2Cl_2$:MeOH:AcOH—9:1:.05.

Example 3
Preparation of 3′,6′-dihydroxy-4′,5′-dimethoxyspiro[isobenzofuran-1(3H),9′-[9H]xanthen]-5 or 6-carboxy-3-one

A mixture of pyrogallol-2-methyl ether (4g) and 1,2,4-benzene tricarboxylic anhydride (2.3g) was heated in an open test tube in a preheated oil bath at 190—195° (outside oil bath temperature) for 3hrs. A dark red mass was obtained. It was cooled and dissolved in 5% NaOH (35ml), and acidified with concentrated HCl to pH1. A dark red oil separated out. The solution was saturated with solid NaCl and stirred in the cold room overnight. The thick black tarry mass was separated and this tar macerated with saturated NaCl solution (2×15ml) TLC ($CH_2Cl_2$: MeOH:AcOH 85:15:1) examination of the tar indicated the presence of a red spot and a few other spots having lower Rf. This tar was absorbed on 5g of RP-2 silica gel60 silanised (E. Marck Cat. No. 7719) and purified by a regular gravity column using RP-2 silica gel (35g) prepared in $CH_2Cl_2$:methanol (100:0.25). The elution was carried out with the same solvent $CH_2Cl_2$:methanol (100:0.25). Elution was followed by TLC. The fractions containing the same red spots were combined and the solvent removed to give a red residue which was macerated with 20ml of a mixture of $CCl_4$:$CH_2Cl_2$ (95:5). The resulting red solid (1.1g) was filtered and was a single spot on TLC. It has $\lambda_{max}^{absorption}$ 512 nm in 0.05m $PO_4^{3-}$ buffer pH8.0 with ε78,200. It has no emission.

Example 4
Preparation of 3′,6′-dihydroxy-4′,5′-dimethoxy-2′,7′-diiodospiro[isobenzofuran-1(3H),9′-[9H]xanthen]-5 or 6-carboxy-3-one

4′,5′-Dimethoxy-6-carboxyfluorescein (Example 3) (1.05g) was dissolved in absolute ethyl alcohol (15ml) and to this was added $I_2$ powder (1g) and solid sodium bicarbonate (0.5g). The contents were refluxed (outside bath temperature 80—85°) for 1hr under $N_2$. TLC examination ($CH_2Cl_2$:methanol:acetic acid 85:14:1) of the refluxing mixture indicated the formation of monoiodinated and small amounts of diiodinated derivatives along with starting material.

More of $I_2$ (500mg) and $NaHCO_3$ (250mg) were added and heating continued for 1hr more. TLC showed formation of more of the mono- and diiodinated products. The addition of more $I_2$ (500mg) and $NaHCO_3$ (250mg) followed by 1hr heating was repeated two more times. TLC of the final mixture indicated the presence of large amounts of diiodinated derivatives along with traces of monoiodinated derivative.

The ethyl alcohol was removed on a Rotovap®. To the residue was added 20ml of water and the solution acidified with dilute HCl to pH1. A brown oil separated out. This was extracted with ether (3×50ml). The dark red ethereal layer was washed with 5% sodium thiosulphate solution (2×20ml) followed by water (1×20ml) and brine solution (1×20ml). The ether was removed on a Rotovap® and the residue dissolved in 4% NaOH (20ml) and filtered. The filtrate was acidified with dilute HCl to give a dark red solid (2.1g).

This red solid (2.1g) was adsorbed on 4g of RP-2 silica gel silanized (E. Merck Cat. No. 9917) using acetone and then poured over a gravity column of RP-2 silica gel (prepared from 30g of RP-2 silica gel using $CH_2Cl_2$) and eluted with $CH_2Cl_2$. Elution was followed by TLC. Fractions having the same Rf were combined and the solvent removed. The residue (~1.1g) was dissolved in 10ml of $CH_2Cl_2$:MeOH (99:1) and n-hexane (7ml) was added. A thick precipitate separated out. This was filtered and the mother liquor concentrated; to which more hexane was added to give more solid. The solids were combined (0.95g) to give 2′,7′-diiodo-4′,5′-dimethoxy-6-carboxylfluorescein as very light-colored long needles which became orange red on standing.

The product has $\lambda_{max}^{absorption}$ 533 nm in 0.05M $PO_4^{3-}$ buffer pH8.0 with ε81,780.

Example 5
Preparation of 9-(2′,5′,6′-trichloro-3′,4′-dicarboxyphenyl)-2,7-dimethoxy-4,5-dichloro-6-hydroxy-3-isoxanthenone

A. Into 25ml of 20% fuming sulfuric acid was dissolved 10g of 4-methylphthalic anhydride and 0.5g powdered iodine. The mixture was heated at 90—100° and chlorine gas was bubbled through the solution continuously. After heating for 24 hrs, an additional 0.5g of iodine was added and the heating continued for an additional 24 hrs. A white solid had precipitated and the solution was cooled, diluted in 100ml ice-cold

water and the white solids filtered. The solid was washed with 20ml cold water and dried *in vacuo*. The product was believed to be a mixture of 3,5,6-trichloro-4-methylphthalic diacid and anhydride.

B. Into a reaction flask was introduced 20g (0.075mole) of the above product and 400ml of aqueous 10% potassium carbonate. After refluxing for about 1hr at 120°, 20ml of *t*-butanol was added, followed by adding 33g (0.2 mole) powdered potassium permanganate in portions to prevent accumulation of the permanganate. An additional 100ml of the 10% aqueous potassium carbonate was used to wash in the permanganate. The reaction course was followed by tlc and when the substantial absence of the starting material was observed, the *t*-butanol was removed by distillation and the resulting slurry acidified with 6M $H_2SO_4$ to pH1. The excess permanganate was destroyed with solid oxalic acid while the pH was maintained at 1 by the addition of sulfuric acid. After concentration *in vacuo*, with formation of a white precipitate, 6M HCl was added to a final volume of 300ml. After stirring for 30min at room temperature, a fine white precipitate formed. The slurry was extracted with ether 3×400ml, the ether solution separated and the ether evaporated *in vacuo*. After azeotropic drying with benzene, 20g of white powder was isolated, which was recrystallized from ethyl acetate-$CCl_4$ to yield 19.5g of the product. mp 238—240°.

C. The above product (10g) was dissolved in 30ml acetic anhydride and heated at 140—145° under nitrogen for 45min. The acetic anhydride was removed *in vacuo* at a temperature of about 35—40°. The white solid weighed 9.5 g. The product is the mixed anhydride with acetic acid and the intramolecular anhydride of the 1,2-dicarboxylic acid groups.

This product (9.5g) can be mixed with 12g of 2-chloro-4-methoxyresorcinol and 1g of anh. zinc chloride and heated at 185—190° for about 1.5hrs. The product is then worked-up as described previously.

Example 6

Conjugation of the product of Example 3 with anti(humanIgG)

A solution of 25mg of the product of Example 3 in 1ml dry THF was stirred with dicyclohexyl carbodiimide (10mg) and 10mg of NHS overnight. After filtering the solution, the solvent was removed and the residue macerated with 10ml of *n*-hexane for 20min. After filtering the resulting solid and washing the solid with 5 ml *n*-hexane, the resulting ester was used without further purification. To a 1% solution of anti(humanIgG) (10 mg protein) in 1ml of 0.05M phosphate buffer, pH8, was slowly added 0.7mg of the above NHS ester in 25µl DMF and the mixture stirred for 1hr at 0—5° and then for an additional hour at room temperature. The product was purified by chromatographing through a G25 Sephadex® column using 0.05M phosphate buffer, pH8. The total resulting volume was 2.2ml. Based on UV absorption at 519nm, the dye/protein ratio was estimated to be about 5.

Example 7

Conjugation of the product of Example 4 with anti(humanIgG)

A. A solution of 4',5'-dimethoxy-2',7'diido-6-carboxyfluorescein (Example 4) (250mg), dicyclohexyl carbodiimide (80mg) and N-hydroxysuccinimide (50mg) in freshly distilled dry THF (3ml) was stirred for 6 hours at room temperature. During this time some white solid separated out which was filtered off. The filtrate was concentrated on a Rotovap® and the residue dried for 5min *in vacuo* to remove the last traces of solvent. The resulting deep red solid was stirred with 12ml of benzene-hexane mixture (1:1) for 20min and the resulting deep violet solid filtered. This solid was found by TLC in $CH_2Cl_2$:MeOH:AcOH (85:15:1) to be almost all NHS ester (as indicated by higher Rf as compared to starting material because the NHS ester reacts with methanol to give the corresponding methyl ester). The violet solid was used as is for conjugation to proteins and is considered to be about 90% pure by weight.

B. To a solution of human-IgG (5mg in 0.5ml of 0.05 M $PO_4^{3-}$ buffer containing 1% cholic acid pH8.0) cooled to 0—5° in an ice-bath was added a solution of the NHS ester prepared above (0.4mg) in 25µm dry DMF slowly during 20min. Continued stirring overnight in the cold room. Next day, the solution was centrifuged for 2 min and the deep red solution purified over a Sephadex® G-25 column (1×30cm) using 0.05M $PO_4^{3-}$ buffer pH8.0 containing 1% cholic acid. The faster moving conjugate was easily separated.

Calculation of the hapten number (i.e., dye/protein ratio) was done as follows. The amount of dye from a UV spectra of a particular dilution is determined by dividing the optical density at 539 by the extinction coefficient 81,780. The amount of protein is determined by knowing the concentration of protein to start with before conjugation and using that concentration number (with appropriate dilution correction for the final conjugate assuming that no protein is lost during conjugation and Sephadex chromatography).

It is found out that there is about 80—90% labelling efficiency of the NHS ester of the subject quencher when labelled according to the above procedure.

Example 8

Preparation of 4,5-dimethoxy-6-hydroxy-9-(2'-carboxyethyl)xanthen-3H-3-one

Into a reaction flask was introduced $0^2$-methyl pyrogallol (300mg), succinic anhydride (100mg) and $ZnCl_2$ (20mg) and the mixture heated at 180—85° for 15min. The product was purified by preparative TLC ($CH_2Cl_2$:MeOH:AcOH:90:10:0.5), and the purified product had $\lambda_{max}^{absorption}$ 506—7nm in 0.05M $PO_4^{3-}$ buffer, pH8.0, with no fluorescence emission.

Following the above procedure, by substituting the succinic anhydride with other anhydrides such as glutaric anhydride, 2,3-dimethylsuccinic anhydride, 1,2-cyclopentanedicarboxylic acid anhydride and adipic anhydride, one would obtain the appropriately substituted 4,5-dimethoxy-6-hydroxy-9-substituted xanthen-3H-3-one.

Example 9

Preparation of 3',6'-dihydroxy-4',5'-dichloro-2',7'-dimethoxyspiro[isobenzofuran-1(3H),9'-[9H]xanthen]-4,7-dichloro-5 or 6-carboxy-3-one

A. 2-Chloro-4-methoxyresorcinol (0.4g) and 3,6-dichlorotrimellitic acid anhydride (0.27g) was heated with $ZnCl_2$ (10mg) at 185° for 15min and the product purified by TLC using $CH_2Cl_2$:MeOH:AcOH::90:10:1. The product had $\lambda_{max}^{absorbance}$ 533nm and $\lambda_{max}^{emission}$ 549—50 nm in 0.05M $PO_4^{3-}$ buffer, pH8.0.

B. The 3,6-dichlorotrimellitic anhydride was prepared as follows:

To a solution of 2,5-dichloro-3,6-dimethylaniline (5g) in dilute HCl (4N, 30ml), maintained at 0° in a three necked flask equipped with a mechanical stirrer, was added dropwise a solution of sodium nitrite (1.85g in 50ml water). After the addition was complete, the diazo solution was neutralized with $Na_2CO_3$ to pH 7 and benzene (100ml) added. To this was then added a freshly prepared solution of cuprous cyanide [prepared from a solution of cuprous chloride and sodium cyanide] with vigorous stirring The mixture was allowed to come to room temperature and then stirred overnight. Next day, the benzene layer was separated and concentrated to give about 4.5g of crude brown solid as the nitrile. This was not purified but hydrolyzed directly to the amide. A solution of the nitrile (4.5g) in a mixture of dioxane (30ml) and 4% aq. NaOH (70ml) was refluxed for 8 hrs. The solution was cooled, extracted with ether and the ethereal solution concentrated to give a brown oil which on maceration with chloroform yielded a brown solid. This was crystallized from chloroform-petroleum ether to give the amide (2.3g).

To a solution of the above amide (0.9g) in conc. $H_2SO_4$ (4ml) was added slowly a solution of sodium nitrite (0.37g in 1 ml of water), while maintaining the temperature of the solution below 30°. After the addition was complete, the mixture was diluted with ice to give a white precipitate which was filtered and further purified by dissolving in 10% $K_2CO_3$ solution, filtering and acidification with 1N HCl to give 2,5-dichloro-3,6-dimethylbenzoic acid.

To the above prepared acid (0.56g) was added alkaline $KMnO_4$ (1.81g in 10ml of 10% $K_2CO_3$) and the mixture heated at 100° for 3 hrs. The resulting mixture was acidified with 6N $H_2SO_4$ to pH1 and excess $KMnO_4$ removed by treatment with oxalic acid. Extraction with ether gave the required triacid product, m.p. 232—33°.

Example 10

Preparation of 2,7-di(methylthio)-6-hydroxy-9-(2'-carboxyphenyl)xanthen-3H-3-one

Into a reaction flask was introduced 4-thiomethylresorcinol (200mg), phthalic anhydride (104mg) and zinc chloride (10mg) and the mixture heated at 180° for 5min. The product was purified by preparative TLC using $CH_3OH$:$CH_2Cl_2$:HOAc (9:1:0.1) and the resulting product had $\lambda_{max}^{absorption}$ 512nm and $\lambda_{max}^{emission}$ 540nm in 0.05 $PO_4^{3-}$ buffer, pH8.0.

In order to demonstrate the utility of the subject compounds, the protein conjugates were employed in an immunoassay as described in U.S. Patent No. 3,996,345. In the first assay a conjugate of fluorescein isothiocyanate and humanIgG having a fluorescein/protein mole ratio of about 7 was employed at a concentration of 0.03mg/ml. The product of Example 6 was employed at a concentration of 4.9mg/ml in 0.05M phosphate buffer, pH8. Varying dilutions of the product of Example 6 were added to a fixed amount of the fluorescein conjugate and incubated for 10min employing as the assay buffer, 0.01M phosphate, 0.15M NaCl and 2% PEG, pH8.0. The protocol followed was to combine 25µl of the fluorescein conjugate in 250µl of buffer with 25µl of the appropriately diluted product of Example 6 in 250µl of buffer, incubate the mixture for 10min, add 2ml of the buffer and then determine the fluorescent signal. When the fluorescein-IgG conjugate was replaced with buffer, so as to obtain background derived from the product of Example 6, the fluorescent signal varied from 0 to 6 with the various dilutions of the product of Example 6, indicating that there was no fluorescence, the observed fluorescence being well within instrumental error.

The following table indicates the results, indicating the various dilutions and the observed fluorescence.

TABLE II

| Dilution of Ex. 6 | Fluorescent signal |
|---|---|
| 1:40 | 722 |
| 1:20 | 598 |
| 1:10 | 382 |
| 1:5 | 198 |
| 1:2.5 | 122 |
| ∞ | 928 |

The above results demonstrate, that the conjugates of the subject invention provide for a large dynamic range in change in fluorescence depending upon the concentration of the quencher conjugate in

relation to the fluorescein present in the medium. The subject data demonstrate that a sensitive assay for gamma-globulin is available, since by adding gamma-globulin to the medium, the amount of available quencher-conjugate for binding to the fluorescer conjugate would be diminished in proportion to the amount of antigen present in the assay medium. Furthermore, by employing the conjugate of the subject invention, background radiation from the subject quencher conjugate is essentially absent.

A second assay was performed, replacing the quencher conjugate of Example 6 with the quencher conjugate of Example 7. The mole ratio of quencher to protein was about 1.5 and the concentration of the quencher solution was approximately 4mg/ml. Instead of fluorescein, the fluorescer employed was 2,7 - dimethyl - 9(2′,5′,6′ - trichloro - 3′,4′ - dicarboxyphenyl) - 6 - hydroxy - 3 - isoxanthenone. This compound was linked through one of the carboxyl groups to IgG through a glycine linking group. The fluorescer/protein mole ratio was about 7.5, and the solution had a concentration of about 0.03mg/ml. The assay described previously was performed with both the quencher of the subject invention, Example 7, and commercially available rhodamineisothiocyanate conjugated to antiIgG. The following table indicates a comparison of the results observed with the two quenchers.

TABLE III

| | Ex. 7 | Rhodamine conjugate |
|---|---|---|
| λ excitation, nm | 520 | 520 |
| λ emission (abs), nm | 550 | 550 |
| Quencher/Protein (mole ratio) | ~1.5 | ~7 |
| Max. Quenching w/1:5 antibody dilution, % | 65—70 | 94 |
| Bkgd. Signal due to Quencher, % of Fluorescer Signal | 6—10 | 60—90 |

(The fluorescer compound described above was prepared from 4-methylresorcinol and 2,5,6-trichloro-1,3,4-phenyltricarboxylic acid anhydride in the manner described previously for the preparation of fluorescein derivatives).

The next assay performed was for the hapten, morphine. 6-Carboxyfluorescein NHS ester was conjugated with $O^3$-(2′-aminoethyl) morphine and the compound of Example 3 conjugated to antimorphine at varying dye/antibody ratios as described in Example 6.

The fluorescent signal from the morphine conjugate was plotted against the concentration of antimorphine conjugate. The assay mixture contained 8.75ng/2.55ml of the fluorescer-morphine conjugate. The antimorphine conjugate was added in amounts varying from 0 to 125µg, with varying ratios of quencher to antimorphine. The buffer employed for the assay mixture 0.01M $PO_4^{3-}$ 0.15M NaCl and 2% PEG 6000. The fluorescein was measured with a Perkin-Elmer 1000 at a concentration of antimorphine-quencher conjugate (based on protein) of 50µg/2.55ml.

| D/P ratio[1] | Fluorescent signal[2] |
|---|---|
| 1 | 715 |
| 3.4 | 400 |
| 7.8 | 110 |
| 10 | 80 |

[1] D/P-quencher/protein mole ratio.
[2] Signal based on 1000 for no antimorphine-quencher conjugate.

By adding morphine to the assay mixture, the fluorescence was enhanced, demonstrating the specificity of the quenching effect.

In comparison to the commonly employed rhodamine as a quencher, the subject quenchers do not have many of the deficiencies of rhodamine and comparable dyes employed as quenchers. Rhodamine has two absorption maxima when conjugated with proteins, with the ratio of the two maxima varying with the degree of labelling. For effective quenching high rhodamine/antibody ratios are required which causes insolubility. This requires the addition of undesirable solubilizing agents e.g. cholic acid or glycerol.

It is evident from the above results that with much lighter labeling than rhodamine, highly efficient

quenching is achieved with the conjugate according to the subject invention. Furthermore, the contribution to background is much smaller than that observed with rhodamine. Thus, one can develop an efficient assay with a high degree of quenching and a minimum amount of interference from light radiated by the quencher.

The quencher conjugate has additional advantages in that it is efficiently conjugated and is quite soluble in the assay medium as compared to rhodamine, which has been previously taught and is conventionally used as a quencher for fluorescein. Furthermore, because of the excellent overlap between the emission of the fluorescer and the absorption of the quencher, high efficiency transfer is achieved. Also, the compounds are easily synthesized and readily conjugated to proteins and other ligands. Finally, precipitation is not observed at the levels of labeling employed so that this problem which was observed with rhodamine is precluded.

It is evident from the above results, that the compositions of the subject invention, both the parent fluorescein derivative quenchers and their conjugates with protein are highly advantageous for use in assays. The compounds are water soluble and can be readily conjugated to proteins. When conjugated to proteins, for example, antibodies, they provide useful reagents in immunoassays where quenching of a fluorescer is necessary. The quenchers of the subject invention provide for highly efficient quenching of the fluorescer, while making only a small or negligible contribution to the amount of light observed from the assay medium.

The fluorescent compounds of this invention absorb at longer wavelengths, so that they can be excited with light which has little, if any, exciting effect on naturally occurring fluorescers encountered in assay samples, e.g. serum. The compounds are water soluble and when conjugated to proteins do not undergo significant changes in the spectroscopic properties of interest. In addition, the compounds have large Stokes shifts, sharp absorption and emission peaks, and good chemical and thermal stability.

## Claims

1. A compound of the formula:

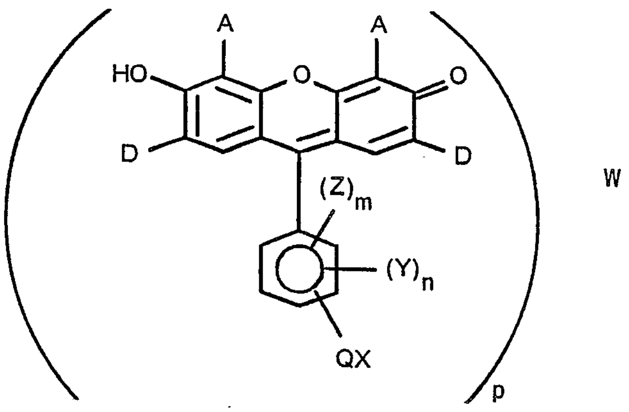

wherein:

the As are the same or different;

the Ds are the same or different;

either the As or the Ds are ethers of the formula —JMX, wherein J is oxygen or sulfur; when other than ethers, they are hydrogen, fluorine, chlorine, bromine, or iodine;

M is a divalent hydrocarbon group of from 1 to 8 carbon atoms;

W is a ligand, receptor, support, or may be taken together with X to form a reactive group for linking;

one of the Xs is a linking group to W; the other X being hydrogen or a group R with the proviso that when X is bonded to a ring carbon atom, X can be halo;

Q is a bond or linking chain;

Y is fluorine, chlorine, bromine or iodine;

Z is an acidic anionic group;

m is an integer of from 0 to 3;

n is an integer of from 0 to 4, wherein m plus n is not greater than 4;

p is at least 1 and up to the molecular weight of W divided by 500;

the group R, when used herein, means the following groups: carboxylic acid, ester, amide, sulfonamide and sulfonic acid.

2. A compound according to Claim 1, wherein the As are alkoxy.

3. A compound according to Claim 1, wherein the Ds are alkoxy.

4. A compound according to any of Claims 2 and 3, wherein W is a poly(amino acid) ligand.

5. A compound according to Claim 1, wherein:

the As are the same and the Ds are the same; and either the As or the Ds are oxyethers of the formula $—OR^bX^b$; when other than $—OR^bX^b$, they are hydrogen, fluorine, chlorine, bromine or iodine;

wherein $R^b$ is saturated aliphatic hydrocarbon of from 1 to 3 carbon atoms;

one of X and $X^b$ is a linking group to W, wherein said linking group includes a carbonyl or sulfonyl; when other than a linking group, $X^b$ is hydrogen or carboxyl and X is hydrogen, carboxyl, fluorine, chlorine, bromine or iodine;

Q is a bond or linking chain, being a bond when X is other than a linking group; when Q is a linking chain, it has a chain of from 1 to 16 atoms which are carbon, sulfur, oxygen and nitrogen, wherein heteroatoms are spaced apart by at least two carbon atoms when bonded to saturated carbon atoms;

Z is a carboxylic acid or sulfonic acid group;

Y is fluorine, chlorine, bromine or iodine;

m is an integer from 1 to 2;

n is an integer from 0 to 4, wherein m plus n is not greater than 4;

W, when not taken together with X or $X^b$ to form a reactive group for linking, is a ligand, receptor or support;

p is 1 when one of X or $X^b$ is taken together with W to form a reactive group for linking, and is otherwise at least 1 and up to the molecular weight of W divided by 500.

6. A compound according to Claim 5, wherein the As are alkoxy of from 1 to 3 carbon atoms.

7. A compound according to Claim 5, wherein the Ds are alkoxy of from 1 to 3 carbon atoms.

8. A compound according to any of Claims 6 or 7, wherein W is a poly(amino acid) and X is a carbonyl.

9. A compound according to Claim 1, wherein:

either the As or the Ds are alkoxy of from 1 to 3 carbon atoms; when other than alkoxy, they are hydrogen, fluorine, chlorine, bromine or iodine;

Z is carboxyl;

m is an integer of from 1 to 2;

Q is a bond or linking chain of from 1 to 12 atoms in the chain, which are carbon, nitrogen and oxygen;

X to which Q is linked is a carbonyl or sulfonyl containing linking group;

W is a ligand of at least about 125 molecular weight, a receptor or a support;

p is at least 1 and up to the molecular weight of W divided by 500.

10. A compound according to Claim 9, wherein said ligand is a hapten of from about 125 to 1000 molecular weight.

11. A compound according to Claim 9, wherein said ligand is an antigen of at least about 5000 molecular weight.

12. A compound according to any of Claims 10 and 11, wherein A is alkoxy of from 1 to 3 carbon atoms, D is hydrogen, fluorine, chlorine, bromine or iodine, Q is a bond or linking group of from 1 to 12 atoms in the chain, X to which Q is linked is carbonyl bonded to an amino group of W to form an amide; and p is about 1 to 100.

13. A compound according to Claim 1, having the formula:

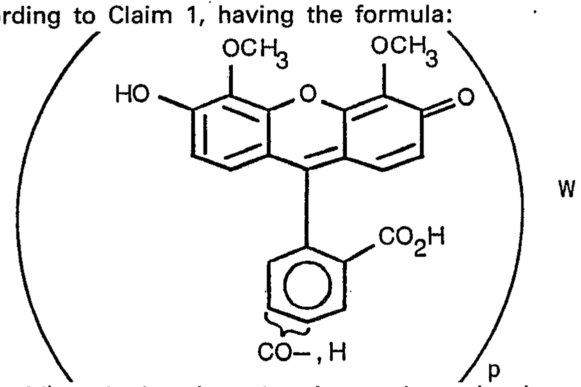

wherein W is a poly(amino acid), and p is at least 1 and up to the molecular weight of W divided by 500.

14. A compound according to Claim 1, having the formula:

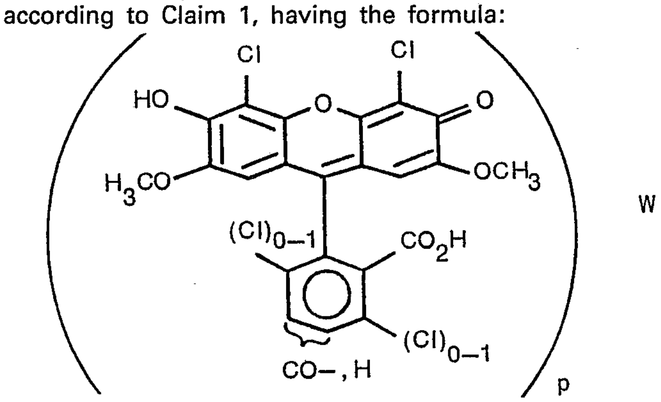

wherein W is a poly(amino acid and p is at least 1 and up to the molecular weight of W divided by 500.

15. A compound according to Claim 1, having the formula:

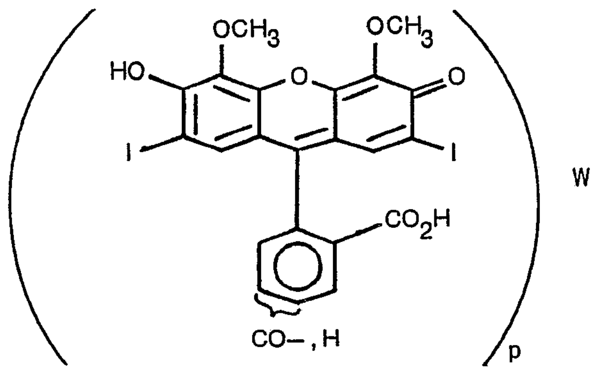

wherein W is a poly(amino acid) and p is at least 1 and up to the molecular weight of W divided by 500.

16. A compound according to Claim 1, having the formula:

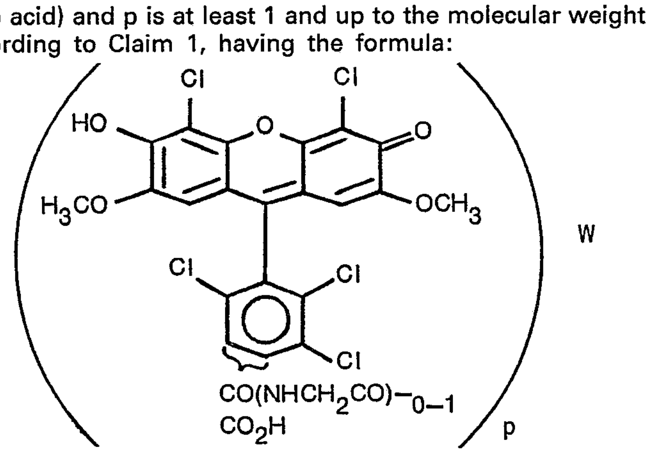

wherein W is a poly(amino acid) and p is at least 1 and up to the molecular weight of W divided by 500.

17. A compound according to Claim 1, having the formula:

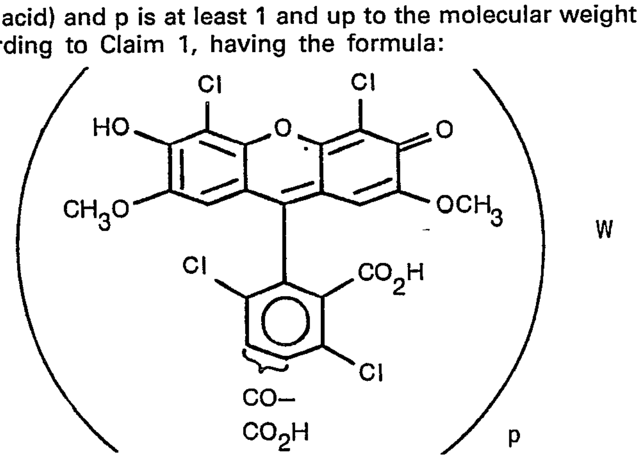

wherein W is a poly(amino acid) and p is at least 1 and up to the molecular weight of W divided by 500.

18. A compound according to any of Claims 13, 14, 15, 16 and 17, wherein said poly(amino acid) is an antigen.

19. A compound according to any of Claims 13, 14, 15, 16 and 17, wherein said poly(amino acid) is an antibody.

20. A compound according to Claim 1, having the formula:

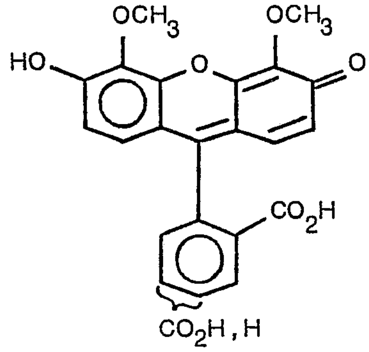

21. A compound according to Claim 1, having the formula:

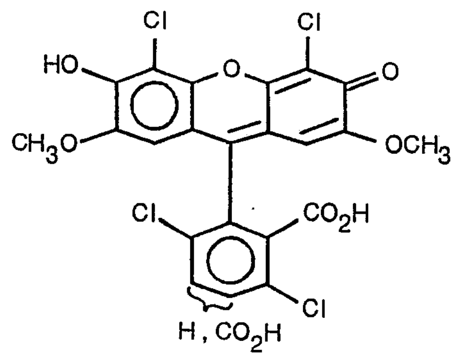

**Patentansprüche**

1. Verbindung der Formel

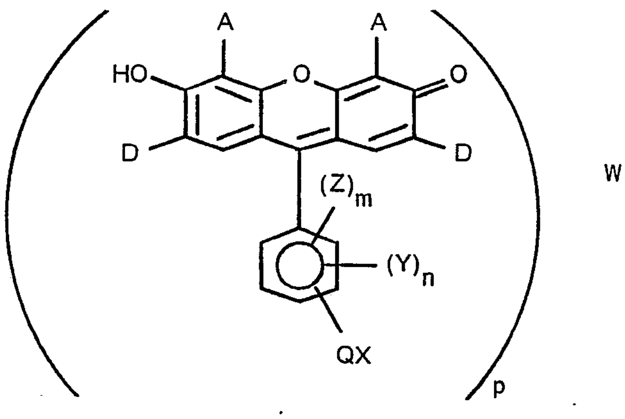

in der

die Substituenten A gleich oder verschieden sind;

die Substituenten D gleich oder verschieden sind;

entweder die Substituenten A oder die Substituenten D Ether der Formel —JMX sind, in der J Sauerstoff oder Schwefel bedeutet; wenn sie keine Ether sind, sind sie Wasserstoff, Fluor, Chlor, Brom oder Jod;

M eine zweiwertige Kohlenwasserstoffgruppe mit 1 bis 8 Kohlenstoffatomen ist;

W ein Ligand, Rezeptor oder Träger ist oder zusammen mit X eine reaktive Gruppe zum Binden sein kann;

einer der Substituenten X eine Bindungsgruppe zu W ist, und der andere Substituent X Wasserstoff oder eine Gruppe R ist mit der Bedingung, daß X Halogen sein kann, wenn X an ein Ringkohlenstoffatom gebunden ist;

Q eine Bindung oder eine Bindungskette ist;

Y Fluor, Chlor, Brom oder Jod ist;

Z eine saure anionische Gruppe ist;

m eine ganze Zahl von 0 bis 3 ist;

n eine ganze Zahl von 0 bis 4 ist, wobei m plus n nicht größer also 4 ist;

p wenigstens 1 ist und bis zum Molekulargewicht von W geteilt durch 500 sein kann;

die Gruppe R die folgenden Gruppen bedeutet: Carbonsäure, Ester, Amid, Sulfonamid und Sulfonsäure.

2. Verbindung nach Anspruch 1, in der die Substituenten A Alkoxygruppen bedeuten.

3. Verbindung nach Anspruch 1, in der die Substituenten D Alkoxygruppen bedeuten.

4. Verbindung nach einem der Ansprüche 2 und 3, in der W ein Polyaminosäure-Ligand ist.

5. Verbindung nach Anspruch 1, in der:

der Substituenten A gleich sind und die Substituenten D gleich sind, und entweder die Substituenten A oder die Substituenten D Oxyether der Formel —$OR^bX^b$ sind; wenn sie nicht —$OR^bX^b$ sind, bedeuten sie Wasserstoff, Fluor, Chlor, Brom oder Jod;

worin $R^6$ ein gesättigter, aliphatischer Kohlenwasserstoff mit 1 bis 3 Kohlenstoffatomen ist;

einer der Substituenten X und $X^b$ eine Bindungsgruppe zu W ist, wobei diese Bindungsgruppe ein Carbonyl oder Sulfonyl einschließt;

wenn X bzw. $X^b$ keine Bindungsgruppe ist, bedeutet $X^b$ Wasserstoff oder Carboxyl und X Wasserstoff, Carboxyl, Fluor, Chlor, Brom oder Jod;

Q eine Bindung oder Bindungskette ist, wobei es eine Bindung ist, wenn X keine Bindungsgruppe ist;

wenn Q eine Bindungskette ist, ist es eine Kette mit 1 bis 16 Atomen, die Kohlenstoff, Schwefel, Sauerstoff und Stickstoff sind, wobei Heteroatome durch mindestens zwei Kohlenstoffatome getrennt sind, wenn sie an gesättigte Kohlenstoffatome gebunden sind;

Z eine Carbonsäure- oder Sulfonsäuregruppe bedeutet;

Y Fluor, Chlor, Brom oder Jod ist;

m eine ganze Zahl von 1 bis 2 ist;

n eine ganze Zahl von 0 bis 4 ist, wobei m plus n nicht größer als 4 ist;

W, wenn es nicht zusammen mit X oder $X^b$ eine reaktive Gruppe zum Binden bildet, ein Ligand, Rezeptor oder Träger ist;

p 1 ist, wenn X oder $X^b$ zusammen mit W eine reaktive Gruppe zum Binden bildet, und andernfalls wenigstens 1 ist und bis zum Molekulargewicht von W geteilt durch 500 sein kann.

6. Verbindung nach Anspruch 5, worin die Substituenten A Alkoxygruppen mit 1 bis 3 Kohlenstoffatomen sind.

7. Verbindung nach Anspruch 5, worin die Substituenten D Alkoxygruppen mit 1 bis 3 Kohlenstoffatomen sind.

8. Verbindung nach einem der Ansprüche 6 oder 7, worin W eine Polyaminosäure und X eine Carbonylgruppe ist.

9. Verbindung nach Anspruch 1, worin:

entweder die Substituenten A oder die Substituenten D Alkoxygruppen mit 1 bis 3 Kohlenstoffatomen sind; wenn sie keine Alkoxygruppen sind, sind sie Wasserstoff, Fluor, Chlor, Brom oder Jod;

Z Carboxyl ist;

m eine ganze Zahl von 1 bis 2 ist;

Q eine Bindung oder eine Bindungskette mit 1 bis 12 Atomen in der Kette ist, die Kohlenstoff, Stickstoff und Sauerstoff sind;

X, an das Q gebunden ist, eine Carbonyl oder Sulfonyl enthaltende Bindungsgruppe ist;

W ein Ligand mit einem Molekulargewicht von wenigstens etwa 125, ein Rezeptor oder ein Träger ist;

p wenigstens 1 ist und bis zum Molekulargewicht von W geteilt durch 500 sein kann.

10. Verbindung nach Anspruch 9, worin der Ligand ein Hapten mit einem Molekulargewicht von etwa 125 bis 1000 ist.

11. Verbindung nach Anspruch 9, worin der Ligand ein Antigen mit einem Molekulargewicht von wenigstens etwa 5000 ist.

12. Verbindung nach einem der Ansprüche 10 und 11, worin A eine Alkoxygruppe mit 1 bis 3 Kohlenstoffatomen, D Wasserstoff, Fluor, Chlor, Brom oder Jod, Q eine Bindung oder Bindungsgruppe mit 1 bis 12 Atomen in der Kette, X, an das Q gebunden ist, Carbonyl, gebunden an eine Aminogruppe von W unter Bildung eines Amids, und p etwa 1 bis 100 ist.

13. Verbindung nach Anspruch 1 mit der Formel

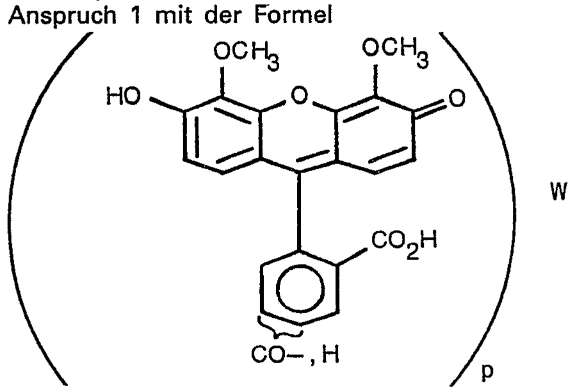

worin W eine Polyaminosäure und p wenigstens 1 ist und bis zum Molekulargewicht von W geteilt durch 500 sein kann.

14. Verbindung nach Anspruch 1 mit der Formel

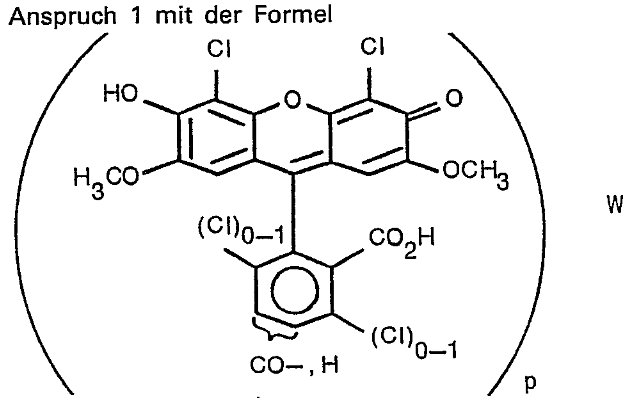

worin W eine Polyaminosäure und p wenigstens 1 ist und bis zum Molekulargewicht von W geteilt durch 500 sein kann.

15. Verbindung nach Anspruch 1 mit der Formel

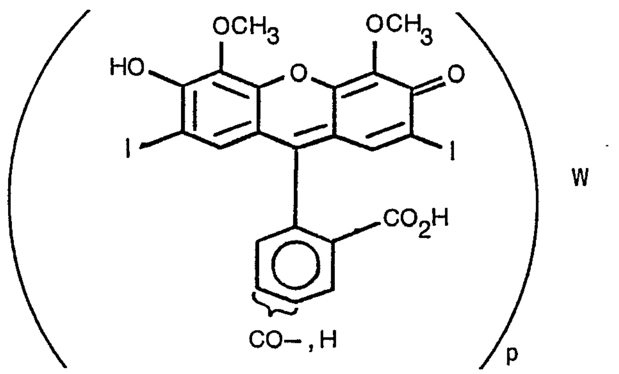

worin W eine Polyaminosäure und p wenigstens 1 ist und bis zum Molekulargewicht von W geteilt durch 500 sein kann.

16. Verbindung nach Anspruch 1 mit der Formel

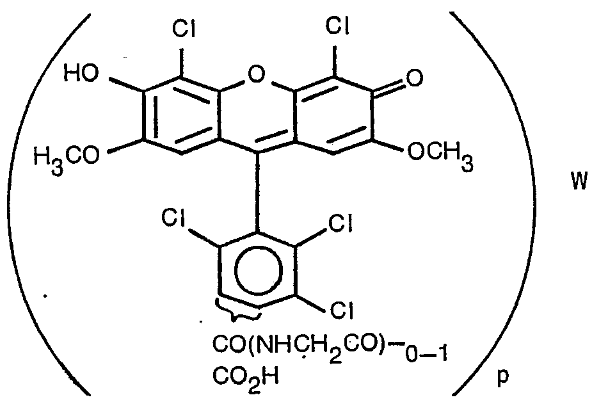

worin W eine Polyaminosäure und p wenigstens 1 ist und bis zum Molekulargewicht von W geteilt durch 500 sein kann.

17. Verbindung nach Anspruch 1 mit der Formel

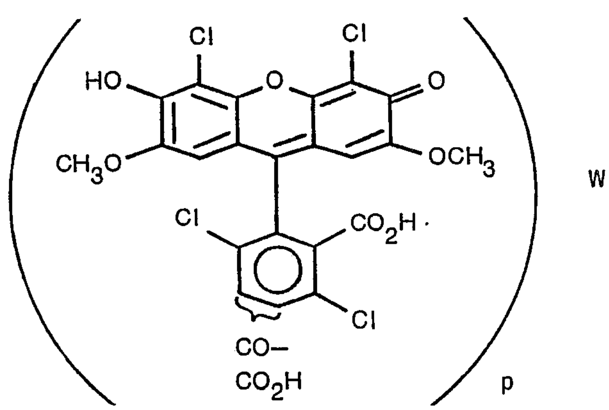

worin W eine Polyaminosäure und p wenigstens 1 ist und bis zum Molekulargewicht von W geteilt durch 500 sein kann.

18. Verbindung nach einem der Ansprüche 13, 14, 15, 16 und 17, worin die Polyaminosäure ein Antigen ist.

19. Verbindung nach einem der Ansprüche 13, 14, 15, 16 und 17, worin die Polyaminosäure ein Antikörper ist.

20. Verbindung nach Anspruch 1 mit der Formel

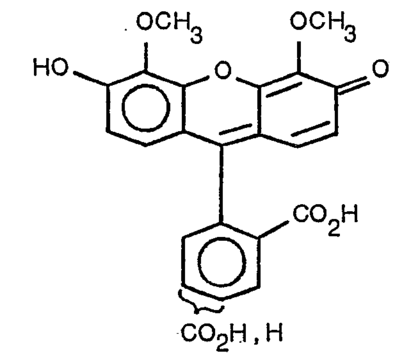

21. Verbindung nach Anspruch 1 mit der Formel

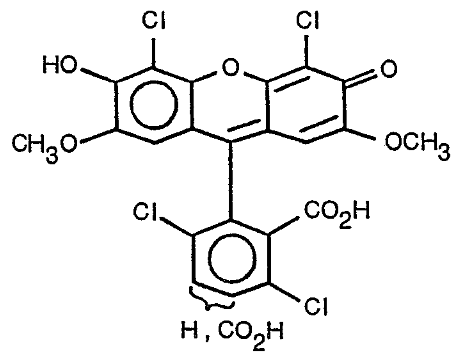

**Revendications**

1. Composé de formule:

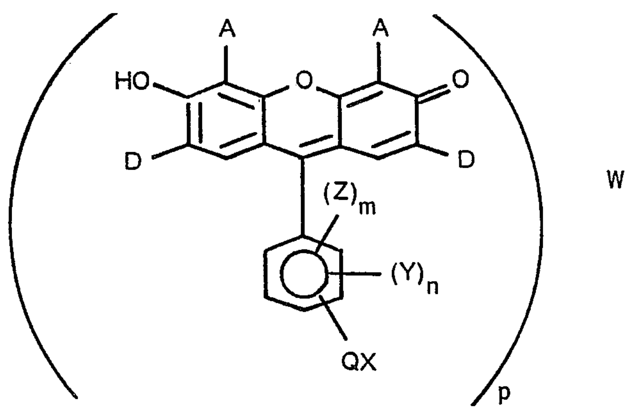

dans laquelle:

les groupes A sont identiques ou différents;

les groupes D sont identiques ou différents;

les groupes A ou D sont des éthers de formule —JMX dans laquelle J est l'oxygène ou le soufre; lorsqu'il s'agit de groupes autres que des éthers, il s'agit d'hydrogène, de fluor, de chlore, de brome ou d'iode;

M est un groupe hydrocarboné divalent ayant 1 à 8 atomes de carbone;

W est un ligand, un récepteur, un support ou peut former conjointement avec X un groupe réactif de jonction;

l'un des groupes X est un groupe de jonction à W; les autres groupes X représentant de l'hydrogène ou un groupe R, sous réserve que lorsque X est attaché à un atome de carbone du noyau, X puisse être aussi un radical halogéno;

Q est un liaison ou un chaîne de jonction;

Y désigne le fluor, le chlore, le brome ou l'iode;

Z est un groupe anionique acide;

m est un nombre entier de 0 à 3;

n est un nombre entier de 0 à 4, la somme m plus n n'étant pas supérieure à 4.

p est au moins égal à 1 et va jusqu'au poids moléculaire de W divisé par 500;

le groupe R, lorsqu'il est utilisé ici, désigne les groupes suivants: acide carboxylique, ester, amide sulfonamide et acide sulfonique.

2. Composé suivant la revendication 1, dans lequel les groupes A sont des groupes alkoxy.

3. Composé suivant la revendication 1, dans lequel les groupes D sont des groupes alkoxy.

4. Composé suivant l'une ou l'autre des revendications 2 et 3, dans lequel W est un ligand poly(aminoacide).

5. Composé suivant la revendication 1, dans lequel:

les groupes A sont identiques et les groupes D sont identiques; et les groupes A ou les groupes D sont des groupes oxyéther de formule $OR^bX^b$; lorsqu'ils ne sont pas des groupes $-OR^bX^b$, il s'agit d'hydrogène, de fluor, de chlore, de brome ou d'iode;

$R^b$ est un hydrocarbure aliphatique saturé de 1 à 3 atomes de carbone;

l'un de X et $X^b$ est un groupe de jonction à W, le groupe de jonction comprenant un groupe carbonyle ou sulfonyl;

lorsqu'il représente autre chose qu'un groupe de jonction, $X^b$ est l'hydrogène ou un groupe carboxyle et X est l'hydrogène, un groupe carboxyle, le fluor, le chlore, le brome ou l'iode;

Q est une liaison ou une chaîne de jonction, représentant une liaison lorsque X représente autre chose qu'un groupe de jonction; lorsque Q est une chaîne de jonction, sa chaîne comprend 1 à 16 atomes qui sont des atomes de carbone, de soufre, d'oxygène et d'azote, les hétéroatomes étant séparés dans cette chaîne par au moins deux atomes de carbone lorsqu'ils sont liées à des atomes saturés de carbone;

Z est un groupe acide carboxylique ou acide sulfonique;

Y représente le fluor, le chlore, le brome ou l'iode;

m est le nombre entier 1 ou 2;

n est un nombre entier de 0 à 4, la somme m plus n ne dépassant pas 4;

W, lorsqu'il n'est pas considéré conjointement avec X ou $X^b$ pour former un groupe réactif de jonction, est un ligand, un récepteur ou un support;

p a la valeur 1 lorsque l'un de X et $X^b$ est pris conjointement avec W pour former un groupe réactif de jonction, sinon il a une valeur allant d'au moins 1 au poids moléculaire de W divisé par 500.

6. Composé suivant la revendication 5, dans lequel les groupes A sont des groupes alkoxy ayant 1 à 3 atomes de carbone.

7. Composé suivant la revendication 5, dans lequel les groupes D sont des groupes alkoxy ayant 1 à 3 atomes de carbone.

8. Composé suivant l'une ou l'autre des revendications 6 et 7, dans lequel W est un poly(aminoacide) et X est un groupe carbonyle.

9. Composé suivant la revendication 1, dans lequel:

les groupes A ou les groupes D sont des groupes alkoxy ayant 1 à 3 atomes de carbone; lorsqu'ils ne sont pas des groupes alkoxy, ils représentent de l'hydrogène, du fluor, du chlore, du brome ou de l'iode;

Z est un groupe carboxyle;

m est le nombre entier 1 ou 2;

Q est une liaison ou une chaîne de jonction de 1 à 12 atomes qui sont des atomes de carbone, d'azote et d'oxygène;

le groupe X auquel Q est attaché est un groupe de jonction contenant un reste carbonyl ou sulfonyle;

W est un ligand de poids moléculaire au moins égal à environ 125, un récepteur ou un support;

p a une valeur allant d'au moins 1 au poids moléculaire de W divisé par 500.

10. Composé suivant la revendication 9, dans lequel ledit ligand est un haptène dont le poids moléculaire va d'environ 125 à 1000.

11. Composé suivant la revendication 9, dans lequel ledit ligand est un antigène de poids moléculaire au moins égal à environ 500.

12. Composé suivant l'une quelconque des revendications 10 et 11, dans lequel A est un groupe alkoxy de 1 à 3 atomes de carbone, D représente l'hydrogène, le fluor, le chlore, le brome ou l'iode, Q est une liaison ou un groupe de jonction ayant 1 à 12 atomes de carbone dans sa chaîne, le groupe X auquel Q est attaché est un groupe carbonyle lié à un groupe amino de W pour former un amide; et p a une valeur d'environ 1 à 100.

13. Composé suivant la revendication 1, de formule:

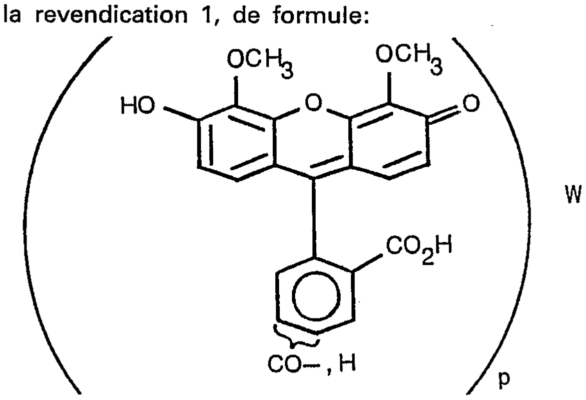

dans laquelle W est un poly(aminoacide) et p a une valeur allang d'au moins 1 au poids moléculaire de W divisé par 500.

14. Composé suivant la revendication 1, de formule:

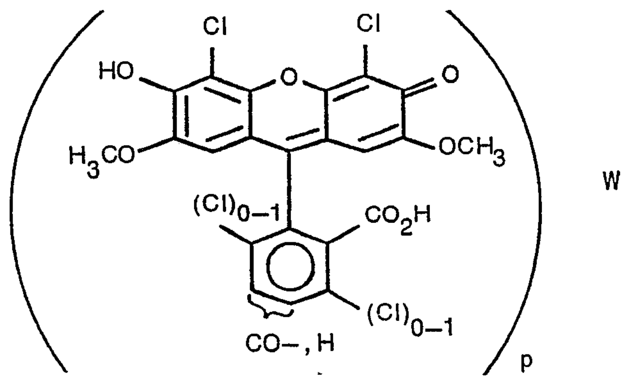

dans laquelle W est un poly(aminoacide) et p a une valeur allant d'au moins 1 au poids moléculaire de W divisé par 500.

15. Composé suivant la revendication 1, de formule:

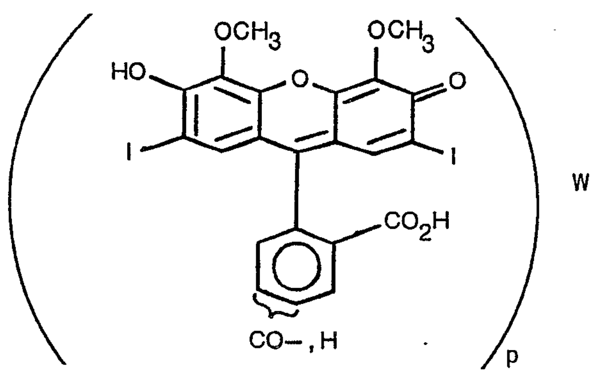

dans laquelle W est un poly(aminoacide) et p a une valeur allant d'au moi1s 1 au poids moléculaire de W divisé par 500.

16. Composé suivant la revendication 1, de formule:

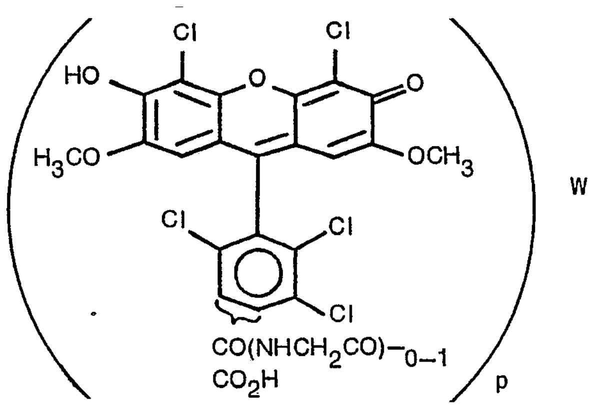

dans laquelle W est un poly(aminoacide) et p a une valeur allant d'au moins 1 au poids moléculaire de W divisé par 500.

17. Composé suivant la revendication 1, de formule:

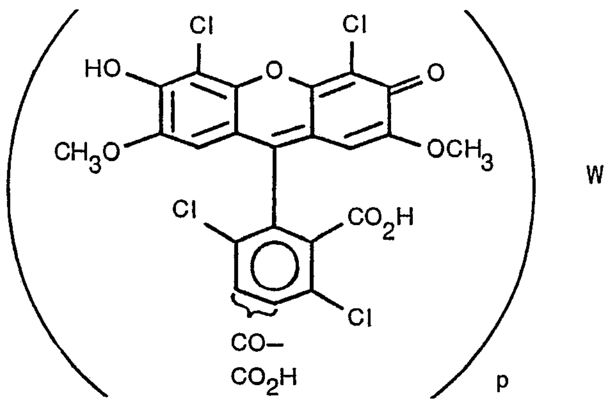

dans laquelle W est un poly(aminoacide) et p a une valeur allant d'au moins 1 au poids moléculaire de W divisé par 500.

18. Composé suivant l'une quelconque des revendications 13, 14, 15, 16 et 17, dans lequel le poly(aminoacide) est un antigène.

19. Composé suivant l'une quelconque des revendications 13, 14, 15, 16 et 17, dans lequel le poly(aminoacide) est un anticorps.

20. Composé suivant la revendication 1, de formule:

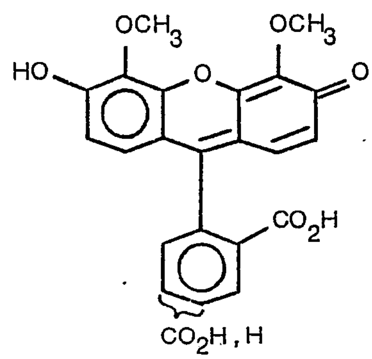

21. Composé suivant la revendication 1, de formule: